# EUROPEAN PATENT APPLICATION

(11) **EP 4 559 407 A1**
(43) Date of publication of application: **28.05.2025**
(21) Application number: 23842929.4
(22) Date of filing: 14.07.2023
(51) Int. Cl.: A61B 10/00, A61B 3/113, G06F 3/01, G06F 3/0346

(54) **STROOP TEST METHOD, STROOP TEST PROGRAM, STROOP TEST SYSTEM, STROOP TEST IMAGE GENERATION METHOD, STROOP TEST IMAGE GENERATION PROGRAM, AND TEST METHOD**

(30) Priority: 21.07.2022 JP 2022116700
(71) Applicant: OSAKA UNIVERSITY, Suita-shi Osaka 565-0871 (JP)
(72) Inventor: TAKEDA, Shuko, Suita-shi, Osaka 565-0871 (JP); MORISHITA, Ryuichi, Suita-shi, Osaka 565-0871 (JP)
(74) Representative: KATZAROV S.A.
(86) International application number: PCT/JP2023/026038
(87) International publication number: WO 2024/019006

(57) **Abstract**

A Stroop test method indicates an evaluation of a subject based on gaze position information indicating a position of a gaze of the subject on a test image (S102 to S105). The test image includes a test problem area that displays a test problem to be given to the subject and a response area that displays a plurality of choices including a correct choice and one or more incorrect choices for selection by the subject to answer the test problem by gazing. The one or more incorrect choices include a distractor choice capable of influencing the subject due to the Stroop effect.

## Description

### [Technical Field]

The present invention relates to a Stroop test method, a Stroop test program, a Stroop test system, a Stroop test image generation method, a Stroop test image generation program, and a test method for executing a Stroop test.

### [Background Art]

Patent Literature (PTL) 1 discloses a cognitive impairment diagnostic device aimed at combining simplicity, low cost, objectivity, quantitativeness, and versatility.

### [Citation List]

### [Patent Literature]

[PTL 1] WO2019/098173

### [Summary of Invention]

### [Technical Problem]

The Stroop test is conventionally known. The Stroop test is a neuropsychological examination that utilizes the Stroop effect and is clinically used as an examination for evaluating the function of the frontal lobe of the brain, which mainly includes attentional function. Here, the Stroop effect is a phenomenon in which two or more pieces of information that a person simultaneously sees, such as the meaning of some text and the color of the text, interfere with each other. It is known that when this information is presented to a person simultaneously with conflicting information, the reaction time until the person selects the information increases, or errors in selecting incorrect information increase (Stroop, 1935).

One representative method of the Stroop test involves the subject responding orally or in writing to questions relating to a character string indicating a color name written on a test sheet or to a color (ink color). In the questions, for example, the subject is instructed to answer the color of the presented ink or to answer the meaning of the presented character string indicating the color name. The examiner calculates the evaluation by measuring the number of answers within the time limit, the number of incorrect answers, and the time required until completion of the Stroop test.

Unfortunately, with conventional Stroop tests, the examiner needs to have a certain level of expertise and training, and must also prepare specialized test sheets, among other things. These requirements contribute to the complexity of preparation, posing a significant barrier to conducting the test. In such a Stroop test, oral responses and scoring based thereon often result in variations from person to person, which is also an issue that may affect the reliability of the evaluation. Stated differently, in such a Stroop test, it is desired that the test can be performed easily and that a quantitative evaluation can be readily obtained.

The present invention has been made in view of the above points and has an object to provide a Stroop test method that can be performed easily and that a quantitative evaluation can be readily obtained.

### [Solution to Problem]

A Stroop test method according to one aspect of the present invention is a method of indicating an evaluation of a subject based on gaze position information indicating a position of a gaze of the subject on a test image. The test image includes: a test problem area that displays a test problem to be given to the subject; and a response area that displays a plurality of choices including a correct choice and one or more incorrect choices for selection by the subject to answer the test problem by gazing. The one or more incorrect choices include a distractor choice capable of influencing the subject due to a Stroop effect.

A Stroop test program according to one aspect of the present invention is for causing a computer to execute: obtaining gaze position information indicating a position of a gaze of a subject on a test image; generating evaluation information indicating an evaluation of the subject based on the gaze position information obtained; and outputting the evaluation information generated. The test image includes: a test problem area that displays a test problem to be given to the subject; and a response area that displays a plurality of choices including a correct choice and one or more incorrect choices for selection by the subject to answer the test problem by gazing. The one or more incorrect choices include a distractor choice capable of influencing the subject due to a Stroop effect.

A Stroop test system according to one aspect of the present invention includes: an obtainer that obtains gaze position information indicating a position of a gaze of a subject on a test image; an evaluator that generates evaluation information indicating an evaluation of the subject based on the gaze position information obtained by the obtainer; and an outputter that outputs the evaluation information generated by the evaluator. The test image includes: a test problem area that displays a test problem to be given to the subject; and a response area that displays a plurality of choices including a correct choice and one or more incorrect choices for selection by the subject to answer the test problem by gazing. The one or more incorrect choices include a distractor choice capable of influencing the subject due to a Stroop effect.

A Stroop test image generation method according to one aspect of the present invention includes: generating a plurality of test images that satisfy a predetermined condition. Each of the plurality of test images includes: a test problem area that displays a test problem to be given to a subject; and a response area that displays a plurality of choices including a correct choice and one or more incorrect choices for selection by the subject to answer the test problem by gazing. The one or more incorrect choices include a distractor choice capable of influencing the subject due to a Stroop effect. The predetermined condition includes that, for a test image among the plurality of test images: (1) the test problem area is positioned at a center of the test image; (2) the response area is positioned in a surrounding area of the test problem area; and (3) the distractor choice in the test image is different from the correct choice in the test image and the correct choice in a test image to be presented next to the subject among the plurality of test images.

A Stroop test image generation program according to one aspect of the present invention is for causing a computer to execute generating a plurality of test images that satisfy a predetermined condition. Each of the plurality of test images includes: a test problem area that displays a test problem to be given to a subject; and a response area that displays a plurality of choices including a correct choice and one or more incorrect choices for selection by the subject to answer the test problem by gazing. The one or more incorrect choices include a distractor choice capable of influencing the subject due to a Stroop effect. The predetermined condition includes that, for a test image among the plurality of test images: (1) the test problem area is positioned at a center of the test image; (2) the response area is positioned in a surrounding area of the test problem area; and (3) the distractor choice in the test image is different from the correct choice in the test image and the correct choice in a test image to be presented next to the subject among the plurality of test images.

A test method according to one aspect of the present invention is a method of indicating an evaluation of a cognitive function of a subject based on gaze position information indicating a position of a gaze of the subject on a test image, and includes: presenting the test image to the subject for a predetermined time. The predetermined time is determined based on a test difficulty level or an age of the subject.

### [Advantageous Effects of Invention]

According to the Stroop test method of the present invention, there is an advantage that the test can be easily performed and a quantitative evaluation can be readily obtained. There is also an advantage that it is possible to objectively and quantitatively measure the degree of hesitation of the subject towards incorrect choices, which could not be evaluated in conventional Stroop tests.

### [Brief Description of Drawings]

[FIG. 1A]

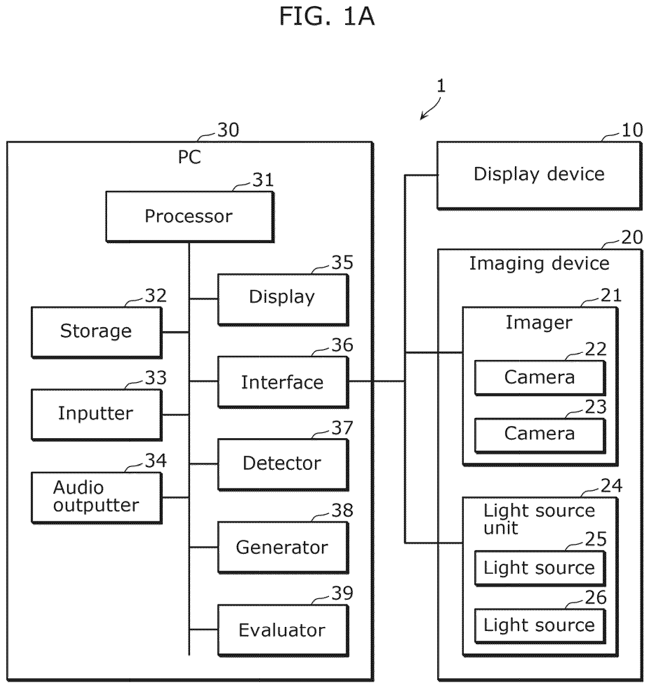

   FIG. 1A is a block diagram illustrating an example configuration of the Stroop test system according to an embodiment of the present disclosure.
[FIG. 1B]

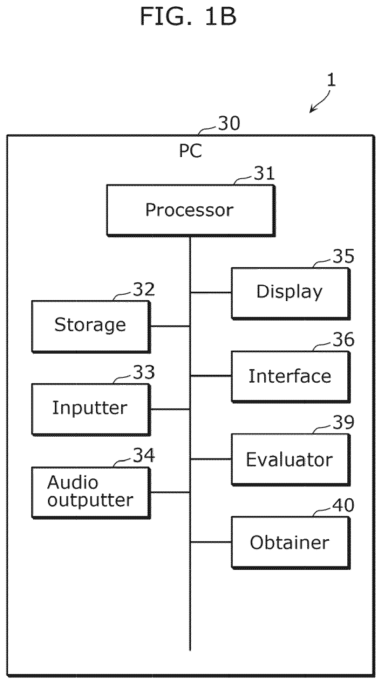

   FIG. 1B is a block diagram illustrating another example configuration of the Stroop test system according to an embodiment of the present disclosure.
[FIG. 2A]

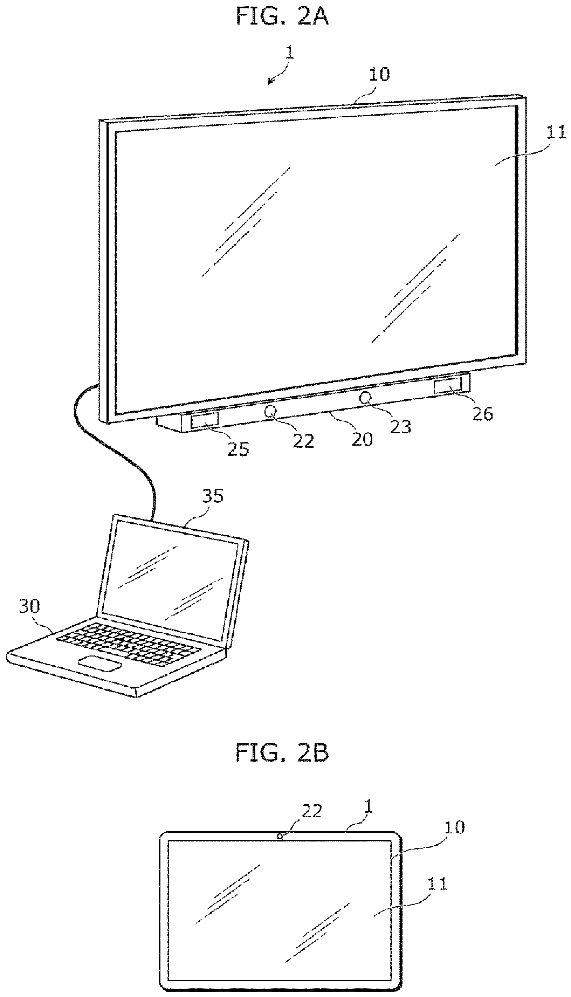

   FIG. 2A is a diagram illustrating an example of an external view of the Stroop test system according to an embodiment of the present disclosure.
[FIG. 2B]
   FIG. 2B illustrates another example of an external view of the Stroop test system according to an embodiment of the present disclosure.
[FIG. 3]

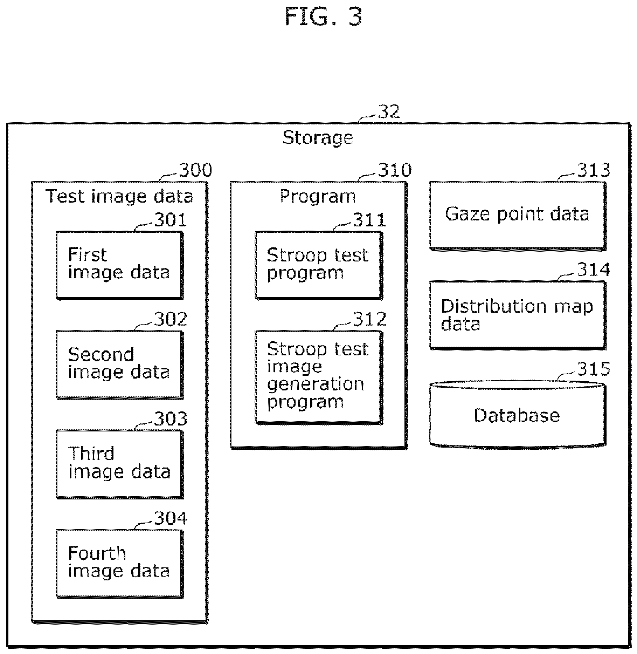

   FIG. 3 illustrates one example of the storage content in a storage unit according to an embodiment of the present disclosure.
[FIG. 4]

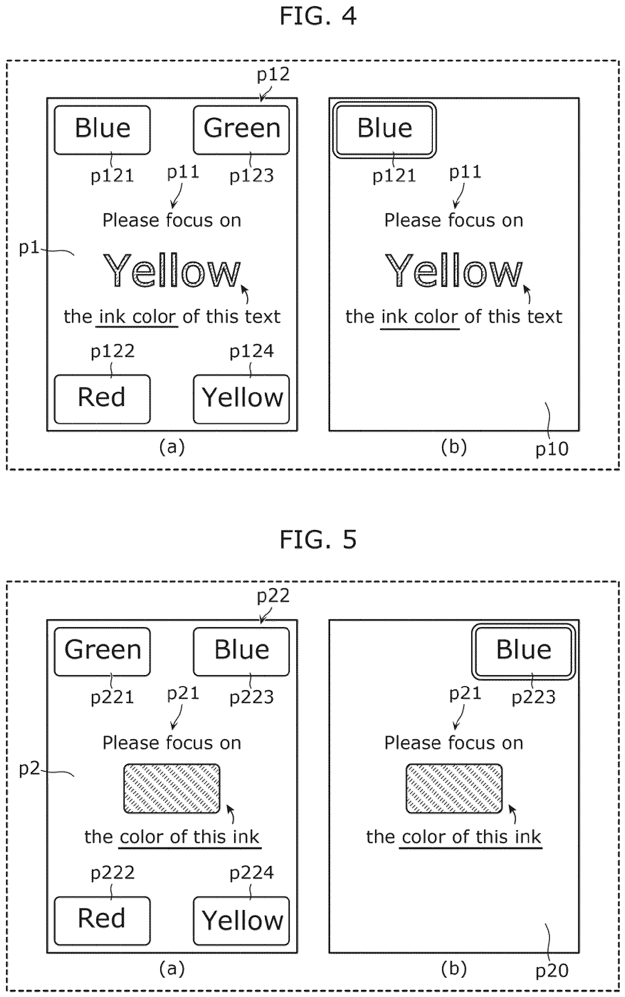

   FIG. 4 illustrates one example of a first image.
[FIG. 5]
   FIG. 5 illustrates one example of a second image.
[FIG. 6]

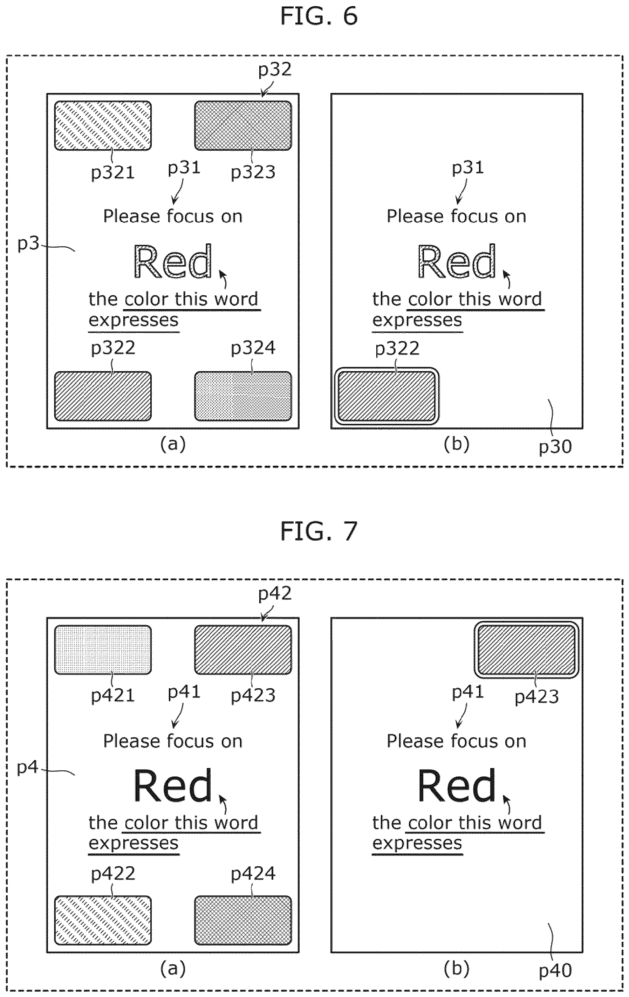

   FIG. 6 illustrates one example of a third image.
[FIG. 7]
   FIG. 7 illustrates one example of a fourth image.
[FIG. 8]

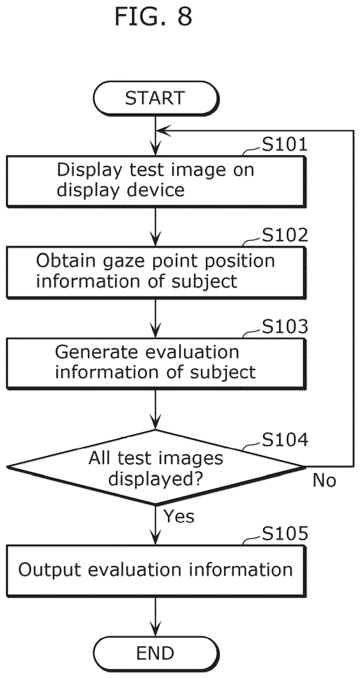

   FIG. 8 is a flowchart illustrating an example of operations performed by the Stroop test system according to an embodiment of the present disclosure.
[FIG. 9]

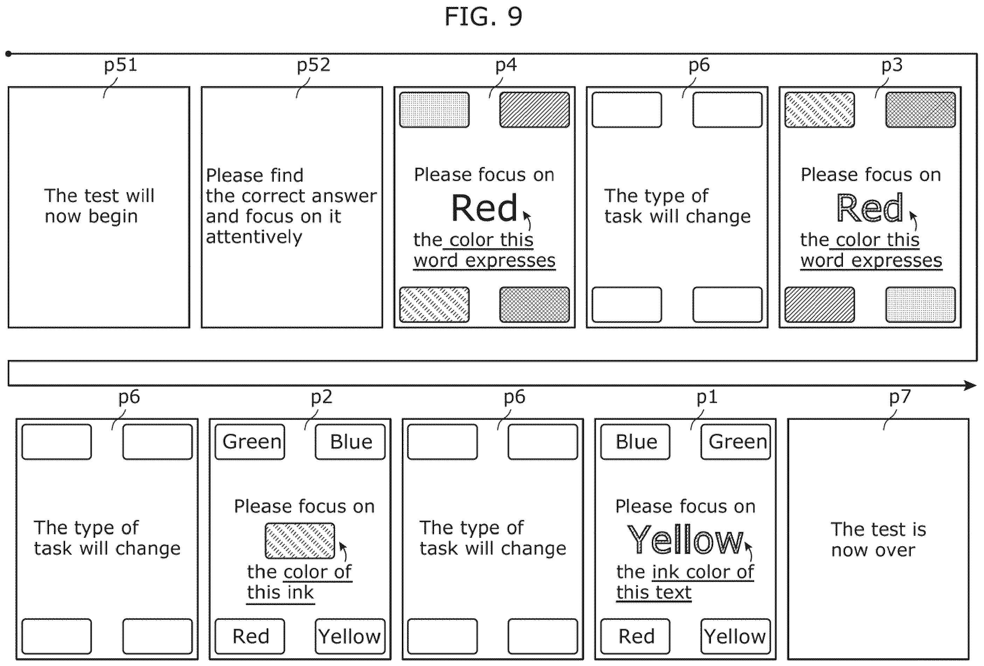

   FIG. 9 illustrates one example of a timeline of images displayed on a display device in the Stroop test according to an embodiment of the present disclosure.
[FIG. 10]

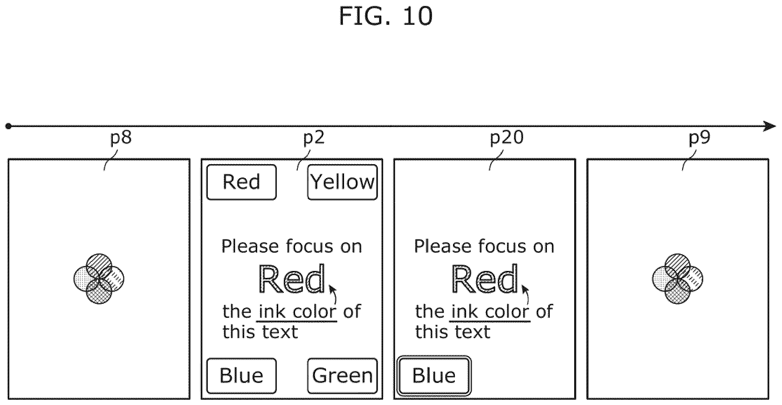

   FIG. 10 illustrates a display example of a test image and a correct answer image.
[FIG. 11]

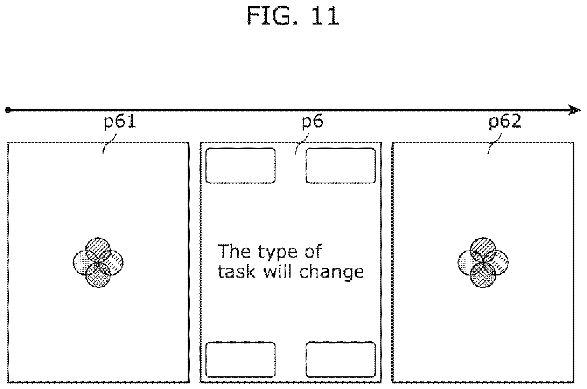

   FIG. 11 illustrates a display example of a transition image.
[FIG. 12]

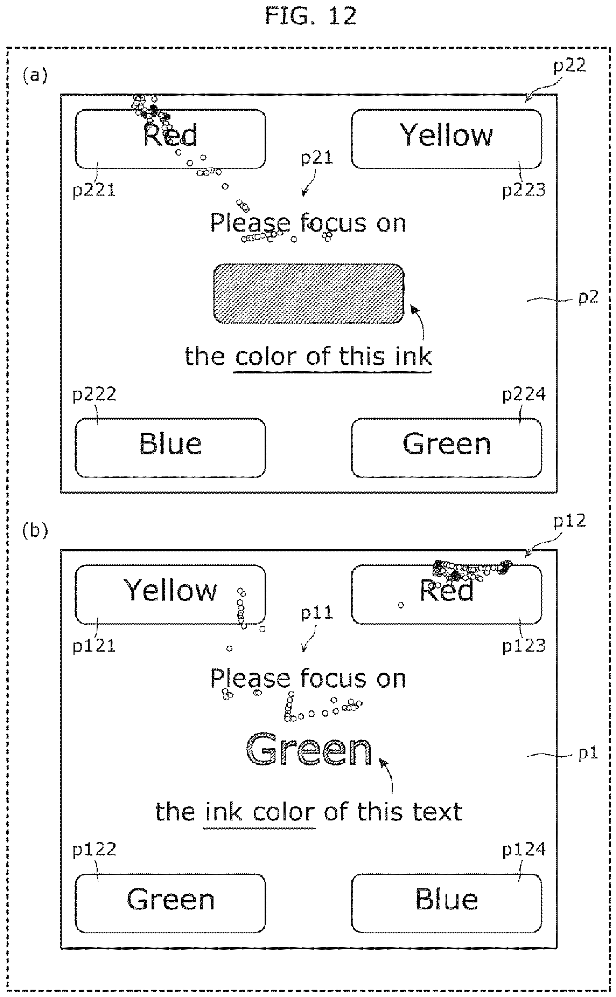

   FIG. 12 illustrates one example of evaluation information of a subject without impairment in cognitive functions including attention.
[FIG. 13]

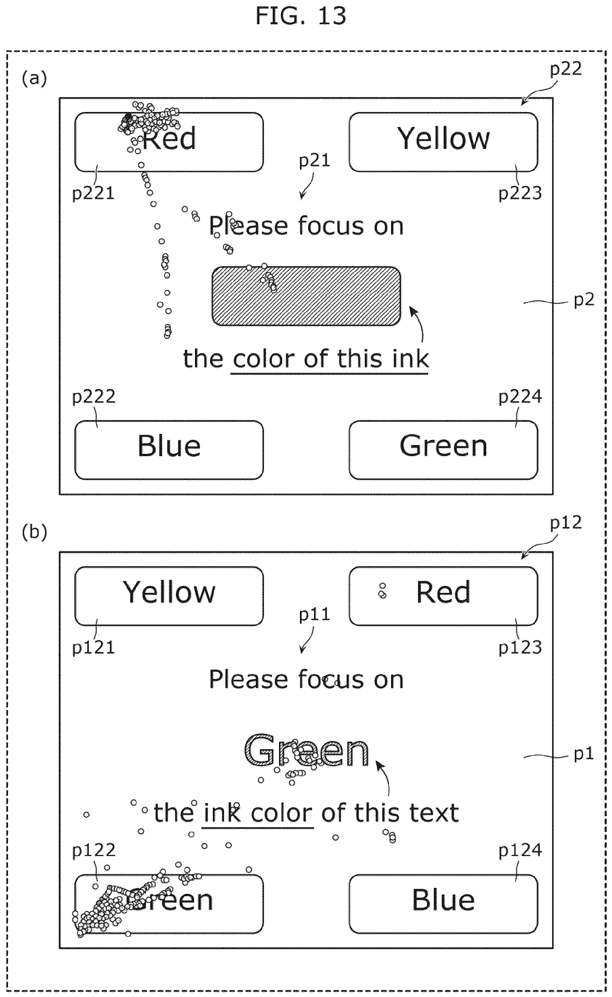

   FIG. 13 illustrates one example of evaluation information of a subject with impairment in cognitive functions including attention.
[FIG. 14]

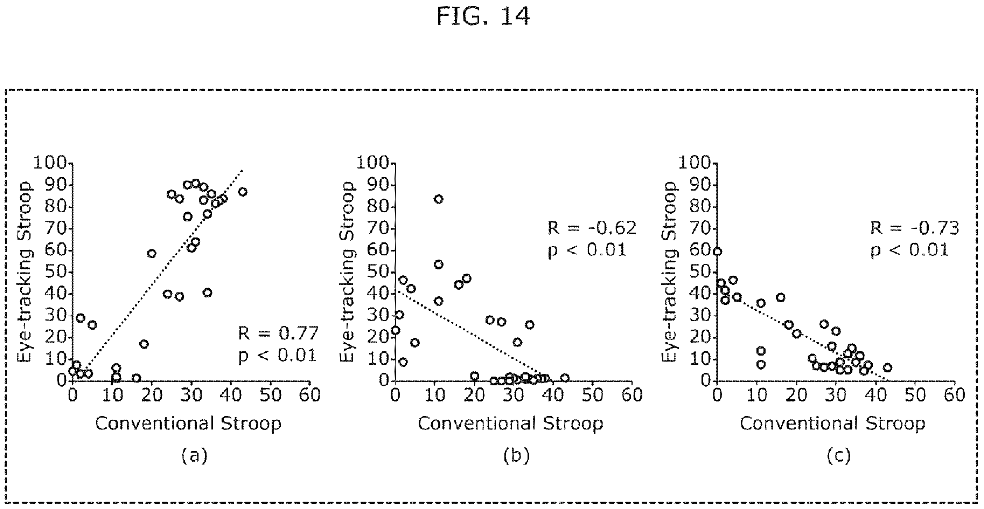

   FIG. 14 is a diagram illustrating a correlation between subject evaluation using the Stroop test system according to an embodiment of the present disclosure and subject evaluation using a conventional Stroop test.
[FIG. 15]

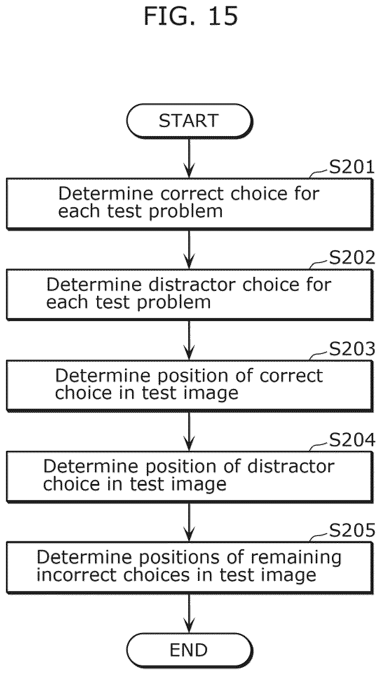

   FIG. 15 is a flowchart illustrating one example of the Stroop test image generation method according to an embodiment of the present disclosure.
[FIG. 16]

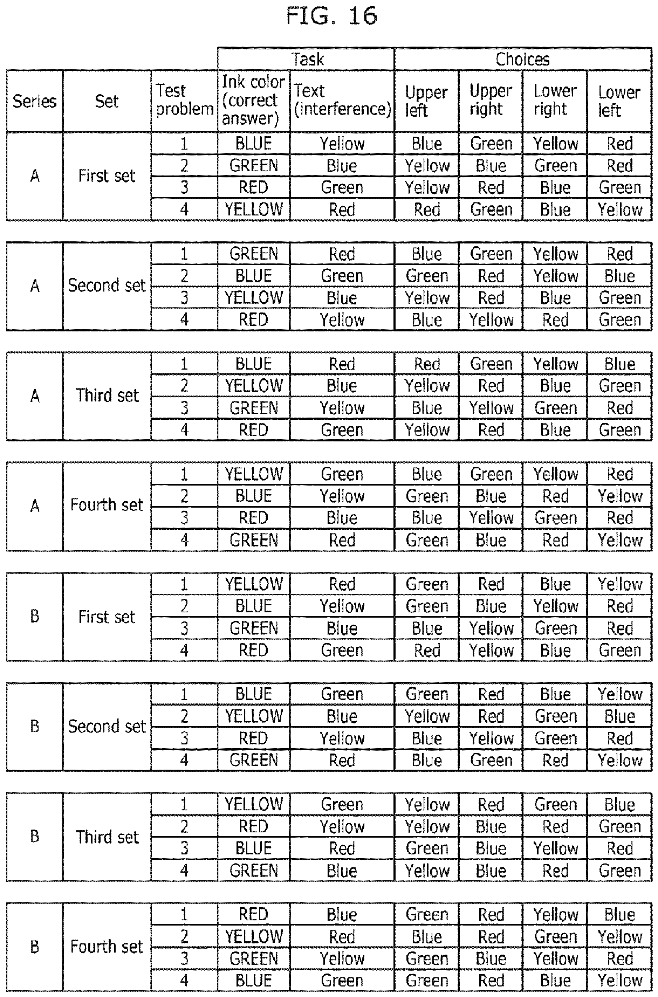

   FIG. 16 illustrates one example of a test image set for a Stroop task generated by the Stroop test image generation method according to an embodiment of the present disclosure.
[FIG. 17]

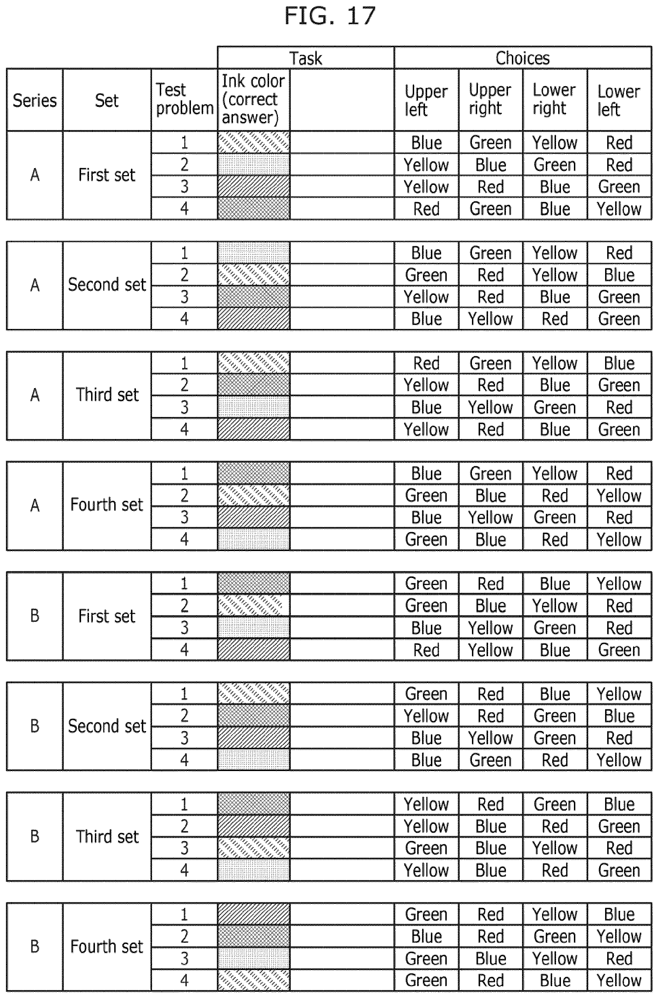

   FIG. 17 illustrates one example of a test image set for a Stroop control task generated by the Stroop test image generation method according to an embodiment of the present disclosure.
[FIG. 18]

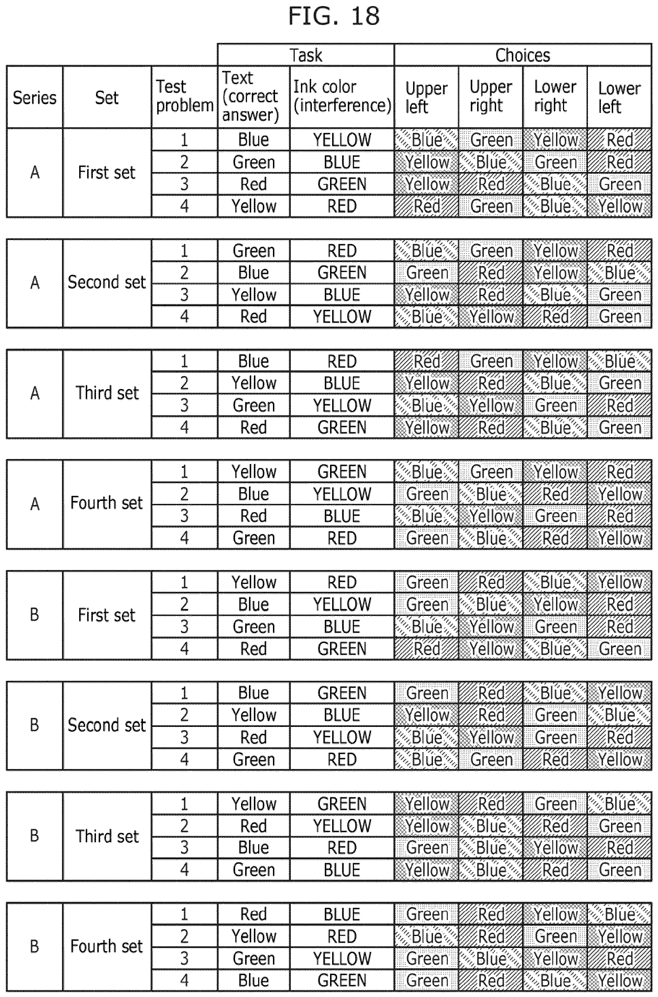

   FIG. 18 illustrates one example of a test image set for a reverse Stroop task generated by the Stroop test image generation method according to an embodiment of the present disclosure.
[FIG. 19]

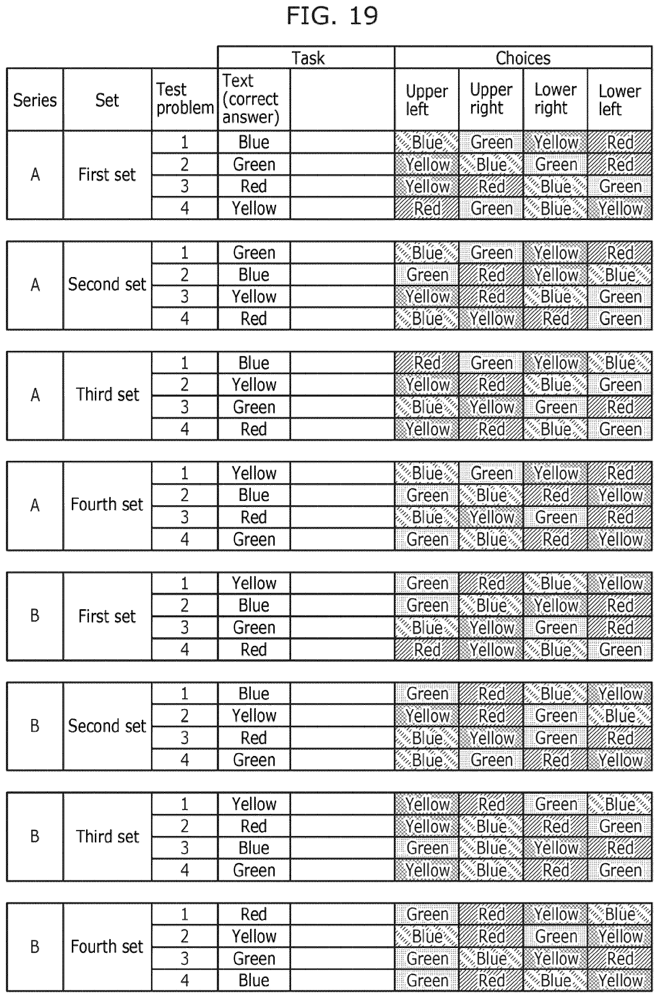

   FIG. 19 illustrates one example of a test image set for a reverse Stroop control task generated by the Stroop test image generation method according to an embodiment of the present disclosure.

### [Description of Embodiments]

### 1. Knowledge Forming the Basis of the Present Invention

First, the point of view of the inventors will be explained.

Conventionally, a cognitive function test (neurophysiological examination) in the form of an interview has been used for evaluation and diagnosis of cognitive impairment and dementia. In such cognitive function tests, the examiner asks the subject questions for evaluating cognitive function, and calculates the subject's cognitive function score by scoring the content of oral or written answers to the questions. One issue with such cognitive function tests is that they are time consuming. For example, even the simplest mini-mental state exam (MMSE) takes about 15 to 30 minutes. Another issue with such cognitive function tests is that the subjects experience significant psychological stress. Furthermore, in such cognitive function tests, the examiner's level of expertise and differences in subjective scoring criteria often affect the calculated cognitive function scores. Due to this, there is also an issue that these factors cause large variations in the cognitive function scores.

To overcome the above issues, the inventors proposed the cognitive impairment diagnostic device of PTL 1.

Attention and judgment are functions primarily handled by the frontal lobe of the brain, and are the most fundamental and important cognitive functions necessary for other cognitive functions such as memory or visuospatial cognition to work normally. As used herein, "cognitive function" is a comprehensive term for brain functions such as attention, judgment, language ability, memory, spatial recognition, etc. A decline in attention and judgment can be observed as a phenomenon associated with fatigue or aging, but it also appears as one of the symptoms in various brain diseases, including dementia. Therefore, the evaluation of attention and judgment is utilized as an important indicator in the diagnosis or assessment of fatigue states, age-related changes in the brain, or brain diseases.

Here, as mentioned in the Technical Problem section, the Stroop test is known as an examination for evaluating the function of the frontal lobe of the brain, which mainly includes attentional function. Conventional Stroop tests include, for example, "New Stroop Test I and II (Hakoda, Watanabe)", "Modified Stroop Test (Perret, 1974)", and "Position Stroop Test", which is one of the tasks in the Clinical Assessment for Attention (CAT). In the Stroop test, it is desired that the test can be performed easily and that a quantitative evaluation can be readily obtained. In conventional Stroop tests, it is difficult to quantitatively measure the degree of hesitation experienced by the subject due to the Stroop effect, and thus a method for objectively and quantitatively measuring this aspect is desired.

In view of the above, the inventors arrived at creating the present invention. Stated differently, the inventors discovered that by combining eye-tracking technology with test images for the Stroop test, it is possible to easily and quantitatively evaluate frontal lobe functions of the brain, such as attention and judgment of the subject.

Hereinafter, the Stroop test method, Stroop test program, Stroop test system, Stroop test image generation method, and Stroop test image generation program according to one or more embodiments will be specifically described with reference to the drawings.

The embodiments described below each illustrate a general or specific example. The numerical values, shapes, materials, elements, the arrangement and connection of the elements, steps, the processing order of the steps etc., shown in the following exemplary embodiment are mere examples, and therefore do not limit the scope of the appended Claims and their equivalents. Therefore, among the elements in the following exemplary embodiment, those not recited in any one of the independent claims are described as optional elements.

The figures are schematic diagrams and are not necessarily precise illustrations. In the figures, elements that are the same share the same reference signs.

### 2. Stroop Test System Configuration

FIG. 1A is a block diagram illustrating an example configuration of the Stroop test system according to the embodiment. FIG. 2A illustrates an example of an external view of the Stroop test system according to the embodiment.

As illustrated in FIG. 1A, Stroop test system 1 includes display device 10, imaging device 20, and personal computer (PC) 30. Here, Stroop test system 1 has a configuration in which a commercially available general PC 30 serves as a primary control device, to which display device 10 and imaging device 20 are further added.

Display device 10 is flat-panel display including display screen 11. Display device 10 displays, on display screen 11, a test image for the Stroop test. Stated differently, in the embodiment, the test image is presented to the subject by being displayed on display device 10. Display device 10 is a large liquid-crystal display or organic electroluminescent (EL) display, as illustrated in FIG. 2A, that is easy even for the elderly to see, for the purpose of showing test images to a subject. Display device 10 may also be a monitor for a personal computer or a television receiver. Instead of a flat-panel display, display device 10 may include a screen and a projector as display screen 11.

Imaging device 20 is a module attachable to display device 10. Imaging device 20 includes at least imager 21 for capturing an image of the eyes of a subject and light source unit 24.

Imager 21 is a stereo camera including camera 22 and camera 23. Camera 22 and camera 23 are each, for example, an infrared camera. As another example, camera 22 and camera 23 may each be a visible light camera. Note that imager 21 need not be a stereo camera, and may be a single camera, or three or more cameras.

Light source unit 24 includes light source 25 and light source 26 for radiating an infrared ray to a subject as illumination light. Each of light source 25 and light source 26 includes, for example, one or more infrared light emitting diodes (LEDs). As another example, light source 25 and light source 26 may each be configured to include one or more white light emitting diodes (LEDs). Note that when the space in which a subject is present is sufficiently bright, imaging device 20 need not include light source unit 24. Imaging device 20 may be attached to an upper portion of display device 10, or cameras 22 and 23 may be separately attached to the left and right sides of display device 10, respectively.

PC 30 includes processor 31, storage 32, inputter 33, audio outputter 34, display 35, interface 36, detector 37, generator 38, and evaluator 39. Of the functional blocks illustrated in FIG. 1A, processor 31, storage 32, inputter 33, audio outputter 34, display 35, and interface 36 are implemented by general hardware and software products of a commercially available computer. The other functional blocks, i.e., detector 37, generator 38, and evaluator 39, are elements that are mainly realized by processor 31 executing the Stroop test program according to the embodiment.

Processor 31 is what is commonly known as a central processing unit (CPU) that executes a program stored in storage 32.

Storage 32 stores programs to be executed by processor 31 and data to be processed by processor 31. Storage 32 further includes database 315. Programs 310 stored in storage 32 include Stroop test program 311 and Stroop test image generation program 312 according to the embodiment in addition to software products such as various firmware, an operating system (OS), and driver software. The data items stored in storage 32 include test image data 300, gaze point data 313, distribution map data 314, etc.

Note that storage 32 includes: a main memory or a primary memory that includes, for example, a dynamic random access memory (DRAM); an auxiliary memory or a secondary memory that includes a hard disc drive (HDD) device or a solid state drive (SSD) device; and a cache memory. Stated differently, storage 32 is a generic name for an element capable of storing programs and data.

Next, the contents stored in storage 32 will be described in detail with reference to FIG. 3. FIG. 3 illustrates one example of the storage content in storage unit 32 according to the embodiment. In FIG. 3, storage 32 stores test image data 300, program 310, gaze point data 313, and distribution map data 314. Program 310 includes Stroop test program 311 and Stroop test image generation program 312. Storage 32 further includes database 315.

Test image data 300 is a still image or a moving image created for Stroop test. Test image data 300 is a set of a plurality of image data items including first image data 301, second image data 302, third image data 303, and fourth image data 304. Each of the plurality of image data items is an image created for conducting a Stroop test. First image data 301 is image data primarily related to the Stroop task, second image data 302 is image data primarily related to the Stroop control task, third image data 303 is image data primarily related to the reverse Stroop task, and fourth image data is image data primarily related to the reverse Stroop control task.

Stroop test program 311 is a program executed by a computer, i.e., PC 30. The Stroop test program causes the computer to execute: obtaining gaze position information indicating the position of the subject's gaze on the test image for the Stroop test; generating evaluation information indicating an evaluation of the subject based on the obtained gaze position information; and outputting the generated evaluation information. In the embodiment, Stroop test program 311 causes the computer to execute: displaying a test image for the Stroop test on display screen 11; capturing, by imager 21, images of the eyes of the subject; detecting a time series of gaze points of the subject on display screen 11, based on the images captured by imager 21; generating a distribution map representing a distribution of the detected gaze points of the subject; generating evaluation information indicating an evaluation of the subject based on the generated distribution map; and displaying the generated evaluation information on display 35.

Of these, the detecting by PC 30 of the time series of gaze points of the subject on display screen 11 based on the images captured by imager 21 is a function of detector 37. The generating by PC 30 of the distribution map representing the distribution of the detected gaze points of the subject is a function of generator 38. The generating by PC 30 of evaluation information indicating an evaluation of the subject based on the generated distribution map is a function of evaluator 39. The displaying by PC 30 of the generated evaluation information on display 35 is a function of display 35.

Stroop test image generation program 312 is a program, executed by a computer, i.e., PC 30, which causes the computer to execute: generating a test image for the Stroop test. Stroop test image generation program 312 will be described in greater detail later in the section "8. Stroop Test Image Generation Method".

Gaze point data 313 is, as will be described later in the explanation of detector 37, time-series data representing the positions and times of the gaze points detected by detector 37, and is a collection of coordinate data (x, y, t) that includes time.

Distribution map data 314 is, as will be described later in the explanation of generator 38, generated by generator 38 by sequentially plotting time-series gaze points in real time on a two-dimensional plane in accordance with the gaze point data, and shows a two-dimensional distribution of the subject's gaze points.

Database 315 stores various data. Note that database 315 need not be a part of storage 32, and may be provided external to storage 32. Database 315 may be connected via a network such as the Internet.

In addition to the program and data illustrated in FIG. 3, storage 32 may also store, for example, diagnostic data indicating the diagnostic result of the subject.

Inputter 33 includes, for example, a keyboard, a mouse, a track pad, etc., and receives an operation from an operator.

Audio outputter 34, one example of which is a loudspeaker, outputs sound.

Display 35 is, for example, a liquid-crystal display. Display 35 displays, for example, a test image superimposed with a distribution map for monitoring by the user (here, the examiner). Display 35 also corresponds to an outputter that outputs the evaluation information generated by evaluator 39.

Interface 36 includes a function for wired communication via, for example, one or more cables connected to display device 10 and imaging device 20. Interface 36 includes, for example, a high-definition multimedia interface (HDMI)^{®} port and a universal serial bus (USB) port. In such cases, interface 36 connects to display device 10 via an HDMI^{®} cable and connects to imager 21 and light source unit 24 via a USB cable. Interface 36 may include, for example, a function for wireless communication with display device 10 and imaging device 20. In this case, the one or more cables are not necessary.

Detector 37 detects the time series of gaze points of the subject on display screen 11 based on the images captured by imager 21. For example, detector 37 detects the eye gaze of the subject from an image captured by imager 21 and detects, as the position of a gaze point on display screen 11, the coordinates of the point at which the eye gaze intersects with display screen 11. Positions of gaze points are detected at regular time intervals. The regular time intervals may be set between tens of milliseconds and hundreds of milliseconds as one example, and thus may be, for example, 100 milliseconds. Detector 37 generates, in real time, a collection of coordinate data (x, y, t) including time, as gaze point data representing a time-series of positions of gaze points of the subject. Here, "x" and "y" are coordinates on a plane (e.g., display screen 11 or cognitive assessment video) and "t" is time.

Generator 38 generates a distribution map representing the distribution of the gaze points of the subject detected by detector 37. Stated differently, generator 38 can also be referred to as an "obtainer" that obtains gaze position information indicating the position of the subject's gaze on the test image. The distribution map is, for example, a map on which marks (e.g., colored dots) corresponding to the above-described coordinate data (x, y, t) are plotted on a two-dimensional plane. The distribution map is superimposed, in real time, on the test image that is displayed on display 35 of PC 30. The marks may be displayed such that, for example, newer gaze points are displayed brighter.

Evaluator 39 generates evaluation information indicating an evaluation of the subject based on the distribution map of the subject generated by generator 38. Stated differently, evaluator 39 generates evaluation information indicating an evaluation of the subject based on the gaze position information of the subject obtained by the obtainer. The evaluation information (in other words, the evaluation) includes, for example, information indicating the degree to which the subject fixated on the correct choice in the test image (here, the fixation rate for the correct choice), information indicating the degree to which the subject fixated on the distractor choice in the test image (here, the fixation rate for the distractor choice), and information indicating the degree to which the subject fixated on the test problem area of the test image (here, the fixation rate for the test problem area). In the embodiment, the evaluation information includes the distribution map of the subject itself.

Note that PC 30 illustrated in FIG. 1A and FIG. 2A may be any one of a laptop computer, a tablet computer, or a desktop computer.

Next, another example configuration of Stroop test system 1 will be given.

FIG. 1B is a block diagram illustrating another example configuration of Stroop test system 1 according to the embodiment. Stroop test system 1 illustrated in FIG. 1B is different from Stroop test system 1 illustrated in FIG. 1A in that it does not include display device 10, imaging device 20, detector 37, and generator 38, and that it further includes obtainer 40. Hereinafter, repeated description of overlapping points will be avoided, and the description will focus on the points of difference.

Display device 10, imaging device 20, detector 37, and generator 38 in FIG. 1A correspond to the elements for eye tracking for generating distribution map data representing the distribution of a subject's gaze points on the test image. In the example configuration illustrated in FIG. 1B, major elements for generating evaluation information indicating an evaluation of the subject based on the subject's gaze position information (in this case, distribution map data) are shown, with the elements for generating the distribution map data in FIG. 1A removed.

Obtainer 40 obtains the distribution map data generated by an external device, and stores it in storage 32. The distribution map data is used in evaluator 39. Note that it is sufficient if obtainer 40 is capable of obtaining at least distribution map data from an external device.

Additionally, it is sufficient if the external device is capable of generating a distribution map representing the distribution of time-series gaze points of a subject by eye tracking. Accordingly, the external device is not limited to a particular eye tracking method or a particular configuration for generating a distribution map. The external device may be connected to communicate with Stroop test system 1 via wired communication, or may be connected to communicate with Stroop test system 1 via wireless communication.

Note that Stroop test system 1 is not required to be a device including a large display device 10 as illustrated in the external view example of FIG. 2A, and may be configured as a smart device.

FIG. 2B illustrates another example of an external view of Stroop test system 1. Stroop test system 1 in FIG. 2B is a tablet smart device having the same functional elements as those illustrated in FIG. 1A, such as display device 10 and imager 21.

Display device 10 illustrated in FIG. 2B doubles as display device 10 and display 35 of PC 30 illustrated in FIG. 2A. A touch panel is provided on the surface of display screen 11.

Imager 21 is a front camera of the smart device. Imager 21 is used, for example, for eye tracking by detecting eye positions and movements using a face recognition technique. In this case, light source unit 24 need not be included. The display of video for a subject is performed by display device 10. In accordance with a touch panel operation performed by the examiner, the Stroop test program or the Stroop test image generation program is executed by the processor inside of the tablet device. The smart device conducts a Stroop test by being alternately used by the subject and the examiner or by the examiner showing display device 10 to the subject.

Stroop test system 1 as the above-described smart device may be implemented by a tablet terminal, a smartphone, or a laptop personal computer. The Stroop test program and the Stroop test image generation program are prepared as apps on these devices. Also note that Stroop test system 1 illustrated in FIG. 2B may include the same functional elements as those illustrated in FIG. 1B.

### 3. Test Image

Next, a test image used in Stroop test system 1 according to the embodiment will be explained. In the embodiment, the test image for the Stroop test is rectangular in shape. In the Stroop test according to the embodiment, the following tests are conducted: a test in which first image p1 (see (a) in FIG. 4) is presented to the subject to obtain a response, a test in which second image p2 (see (a) in FIG. 5) is presented to the subject to obtain a response, a test in which third image p3 (see (a) in FIG. 6) is presented to the subject to obtain a response, and a test in which fourth image p4 (see (a) in FIG. 7) is presented to the subject to obtain a response.

In FIG. 4 through FIG. 7, red is represented by solid line hatching, blue is represented by broken line hatching, green is represented by dot hatching, and yellow is represented by diamond-shaped hatching. This representation of colors is the same in subsequent figures as well.

FIG. 4 illustrates one example of first image p1. In FIG. 4, (a) illustrates first image p1, and in FIG. 4, (b) illustrates correct answer image p10 for first image p1. First image p1 is a test image to be presented to the subject to obtain a response regarding the Stroop task. The Stroop task is a task for confirming the influence of the Stroop effect on the subject, and is particularly a task for confirming the degree to which the subject is strongly affected by interference from text. First image p1 includes test problem area p11 and response area p12.

Test problem area p11 is an area where a problem to be given to the subject is displayed, and is located at the center of first image p1 (i.e., the test image). In the example shown in (a) in FIG. 4, test problem area p11 displays a character string indicating a color name (here, "yellow"), a test problem sentence regarding the ink color used (here, blue) for the character string (here, "Please focus on the ink color of this text"). A character string indicating a color name means an ink color different from the correct ink color.

Response area p12 is an area where a plurality of choices (here, four choices p121 to p124) for selection by the subject to answer the question are displayed, and is located at each of a plurality of corners (here, four corners) of first image p1 (i.e., the test image). The plurality of choices include a correct choice indicating the correct answer to the test problem given to the subject, and one or more incorrect choices indicating incorrect answers to the test problem given to the subject. The one or more incorrect choices include a distractor choice that may influence the subject due to the Stroop effect. The distractor choice, in other words, is the choice among the one or more incorrect choices that most strongly interferes with the correct choice.

In this way, by placing the test problem area at the center of the test image and placing the plurality of choices at respective corners of the test image, there is an advantage that when the subject's gaze moves toward each choice after fixating on the test problem area, the trajectory of the subject's gaze is more likely to be dispersed, and the trajectories of the subject's gaze toward each choice are less likely to overlap. This point is important in terms of increasing the accuracy of evaluation of the subject in the Stroop test method according to the embodiment which calculates the fixation rate for each choice from gaze information.

In the example illustrated in (a) in FIG. 4, the correct choice is choice p121, which is the character string indicating the correct ink color (here, "blue"), and is located at the upper left corner of first image p1. In the example illustrated in (a) in FIG. 4, the one or more incorrect choices are the three choices p122 to p124, which are character strings indicating incorrect ink colors (here, "red", "green", and "yellow"), and are located at the three corners of first image p1 excluding the upper left corner. In the example illustrated in (a) in FIG. 4, the distractor choice is choice p124, which is the character string indicating the color name in test problem area p11 (here, "yellow"), and is located at the lower right corner of first image p1. Stated differently, the correct choice and the distractor choice are located diagonally opposite each other across test problem area p11.

By arranging the correct choice and the distractor choice in this way, it is possible to clearly distinguish between the correct choice and the distractor choice, and there is an advantage that even if there is a deviation in the subject's gaze, it becomes easier to distinguish and detect whether the subject's gaze is directed toward the correct choice or toward the distractor choice.

Correct answer image p10 for first image p1 is an image for presenting the correct answer for the Stroop task to the subject, and as illustrated in (b) in FIG. 4, includes test problem area p11 similar to that of first image p1, and the correct choice (here, choice p121). In correct answer image p10, the correct choice may be emphasized by, for example, being enclosed by a relatively thick line or the like.

FIG. 5 illustrates one example of second image p2. In FIG. 5, (a) illustrates second image p2, and in FIG. 5, (b) illustrates correct answer image p20 for second image p2. Second image p2 is a test image to be presented to the subject to obtain a response regarding the Stroop control task. The Stroop control task is a task for confirming whether the subject has the ability to answer questions in the Stroop task, prior to presenting the Stroop task to the subject. Here, the Stroop control task is a task for confirming whether the subject can recognize the color of ink. Second image p2 includes test problem area p21 and response area p22.

Test problem area p21 is an area where a test problem to be given to the subject is displayed, and is located at the center of second image p2 (i.e., the test image). In the example shown in (a) in FIG. 5, test problem area p21 displays a rectangular region filled with the correct ink color (here, blue), and a test problem sentence regarding the ink color used (here, blue) in the rectangular region (here, "Please focus on the color of this ink").

Response area p22 is an area where a plurality of choices (here, four choices p221 to p224) for selection by the subject to answer the question are displayed, and is located at each of a plurality of corners (here, four corners) of second image p2 (i.e., the test image). The plurality of choices include a correct choice indicating the correct answer to the test problem given to the subject, and one or more incorrect choices indicating incorrect answers to the test problem given to the subject. Note that in the Stroop control task, the one or more incorrect choices do not include a distractor choice.

In the example illustrated in (a) in FIG. 5, the correct choice is choice p223, which is the character string indicating the correct ink color (here, "blue"), and is located at the upper right corner of second image p2. In the example illustrated in (a) in FIG. 5, the one or more incorrect choices are the three choices p221, p222, and p224, which are character strings indicating incorrect ink colors (here, "green", "red", and "yellow"), and are located at the three corners of second image p2 excluding the upper right corner.

Correct answer image p20 for second image p2 is an image for presenting the correct answer for the Stroop control task to the subject, and as illustrated in (b) in FIG. 5, includes test problem area p21 similar to that of second image p2, and the correct choice (here, choice p223). In correct answer image p20, the correct choice may be emphasized by, for example, being enclosed by a relatively thick line or the like.

FIG. 6 illustrates one example of third image p3. In FIG. 6, (a) illustrates third image p3, and in FIG. 6, (b) illustrates correct answer image for third image p3. Third image p3 is a test image to be presented to the subject to obtain a response regarding the reverse Stroop task. The reverse Stroop task is a task for confirming the influence of the Stroop effect on the subject, and is particularly a task for confirming the degree to which the subject is strongly affected by interference from color. Third image p3 includes test problem area p31 and response area p32.

Test problem area p31 is an area where a test problem to be given to the subject is displayed, and is located at the center of third image p3 (i.e., the test image). In the example shown in (a) in FIG. 6, test problem area p31 displays a character string indicating a color name (here, "red"), and a test problem sentence regarding the meaning (here, red) of the character string (here, "Please focus on the color this word expresses"). A character string indicating a color name is represented in an ink color (in this case, blue) different from the correct ink color.

Response area p32 is an area where a plurality of choices (here, four choices p321 to p324) for selection by the subject to answer the question are displayed, and is located at each of a plurality of corners (here, four corners) of third image p3 (i.e., the test image). The plurality of choices include a correct choice indicating the correct answer to the test problem given to the subject, and one or more incorrect choices indicating incorrect answers to the test problem given to the subject. The one or more incorrect choices include a distractor choice that may influence the subject due to the Stroop effect.

In the example illustrated in (a) in FIG. 6, the correct choice is choice p322, which is a rectangular region filled with the correct ink color (here, red), and is located at the lower left corner of third image p3. In the example illustrated in (a) in FIG. 6, the one or more incorrect choices are the three choices p321, p323, and p324, which are rectangular regions filled with incorrect ink colors (here, blue, yellow, and green), and are located at the three corners of third image p3 excluding the lower left corner. In the example illustrated in (a) in FIG. 6, the distractor choice is choice p321, which is a rectangular region filled with the color used for the character string indicating the color name in test problem area p31 (here, blue), and is located at the upper left corner of third image p3. Note that in the example illustrated in (a) in FIG. 6, the correct choice and the distractor choice are not located diagonally opposite each other across test problem area p31, but they may be located diagonally opposite each other across test problem area p31.

Correct answer image p30 for third image p3 is an image for presenting the correct answer for the reverse Stroop task to the subject, and as illustrated in (b) in FIG. 6, includes test problem area p31 similar to that of third image p3, and the correct choice (here, choice p322). In correct answer image p30, the correct choice may be emphasized by, for example, being enclosed by a relatively thick line or the like.

FIG. 7 illustrates one example of fourth image p4. In FIG. 7, (a) illustrates fourth image p4, and in FIG. 7, (b) illustrates correct answer image p40 for fourth image p4. Fourth image p4 is a test image to be presented to the subject to obtain a response regarding the reverse Stroop control task. The reverse Stroop control task is a task for confirming whether the subject has the ability to answer questions in the reverse Stroop task, prior to presenting the reverse Stroop task to the subject. Here, the reverse Stroop control task is a task for confirming whether the subject can recognize the meaning represented by the character string. Fourth image p4 includes test problem area p41 and response area p42.

Test problem area p41 is an area where a test problem to be given to the subject is displayed, and is located at the center of fourth image p4 (i.e., the test image). In the example shown in (a) in FIG. 7, test problem area p41 displays a character string indicating a color name (here, "red"), and a test problem sentence regarding the meaning (here, red) of the character string (here, "Please focus on the color this word expresses"). The ink color of the character string indicating a color name is an ink color (in this case, black) different from the ink colors that the subject can select.

Response area p42 is an area where a plurality of choices (here, four choices p421 to p424) for selection by the subject to answer the question are displayed, and is located at each of a plurality of corners (here, four corners) of fourth image p4 (i.e., the test image). The plurality of choices include a correct choice indicating the correct answer to the test problem given to the subject, and one or more incorrect choices indicating incorrect answers to the test problem given to the subject. Note that in the reverse Stroop control task, the one or more incorrect choices do not include a distractor choice.

In the example illustrated in (a) in FIG. 7, the correct choice is choice p423, which is a rectangular region filled with the correct ink color (here, red), and is located at the upper right corner of fourth image p4. In the example illustrated in (a) in FIG. 7, the one or more incorrect choices are the three choices p421, p422, and p424, which are rectangular regions filled with incorrect ink colors (here, green, blue, and yellow), and are located at the three corners of fourth image p4 excluding the upper right corner.

Correct answer image p40 for fourth image p4 is an image for presenting the correct answer for the reverse Stroop control task to the subject, and as illustrated in (b) in FIG. 7, includes test problem area p41 similar to that of fourth image p4, and the correct choice (here, choice p423). In correct answer image p40, the correct choice may be emphasized by, for example, being enclosed by a relatively thick line or the like.

### 4. Operations

Next, an example of operations performed by Stroop test system 1 according to the embodiment, i.e., one example of the Stroop test method according to the embodiment, will be given with reference to FIG. 8. FIG. 8 is a flowchart illustrating an example of operations performed by Stroop test system 1 according to the embodiment. The Stroop test processes illustrated in FIG. 8 are processes mainly realized by PC 30 executing Stroop test program 311. Note that a calibration process for gaze point detection may be performed before executing the Stroop test process illustrated in FIG. 8.

First, PC 30 executes a process of displaying a test image for the Stroop test on display device 10 (S101). Next, PC 30 obtains the subject's gaze position information for the test image displayed on display device 10 (S102). In step S102, for example, PC 30 illustrated in FIG. 1A detects the position of the subject's gaze point in the test image by detector 37, and obtains the subject's gaze position information by generator 38 generating the subject's distribution map for the test image. In step S102, for example, PC 30 illustrated in FIG. 1B obtains the subject's gaze position information from an external device via obtainer 40.

PC 30 generates the subject's evaluation information based on the obtained gaze position information (in other words, the generated distribution map) (S103). In step S103, PC 30 sets the test problem area and response area in the test image as regions of interest (ROI), and generates evaluation information by calculating the fixation rate in the regions of interest. Here, the fixation rate is a parameter expressed as a percentage representing the ratio of the number of the subject's gaze points in the region of interest to the total number of the subject's gaze points during the time the test image is displayed. Here, PC 30 calculates the fixation rate for the test problem area, the fixation rate for the correct choice, and the fixation rate for the distractor choice.

Subsequently, PC 30 repeatedly executes the above steps S101 to S103 (S104: No) until all test images are displayed on display device 10. Stated differently, when there are a plurality of test images, PC 30 sequentially displays the test images on display device 10 at predetermined time intervals, and executes the above steps S101 to S103 each time a test image is displayed on display device 10. When PC 30 has displayed all test images on display device 10 (S104: Yes), it outputs the evaluation information by displaying the generated evaluation information on display 35 (S105). Note that step S105 may be executed each time evaluation information is generated in step S103.

### 5. Specific Example of Stroop Test

Here, specific examples of the Stroop test according to the embodiment will be given with reference to FIG. 9 through FIG. 11. FIG. 9 illustrates one example of a timeline of images displayed on display device 10 in the Stroop test according to the embodiment. FIG. 10 illustrates a display example of a test image and a correct answer image. FIG. 11 illustrates a display example of a transition image.

As illustrated in FIG. 9, PC 30 first sequentially displays start images p51 and p52 on display device 10 to notify the subject of the start of the Stroop test. Start image p51 includes a character string (in this example, "The test will now begin") in the center to notify the subject of the start of the Stroop test. Moreover, start image p52 includes a character string (in this example, "Please find the correct answer and focus on it attentively") in the center to explain how to respond in the Stroop test. The display time of start image p51 is, for example, approximately 2.5 seconds, and the display time of start image p52 is, for example, approximately 2.5 seconds.

Next, PC 30 displays a test image (fourth image p4) for the reverse Stroop control task on display device 10. Here, PC 30 sequentially displays a plurality of test problem sets (for example, 4 test problem sets) on display device 10, with each test problem set including fourth image p4 and correct answer image p40. The display time of one test problem set is, for example, approximately 8 seconds or 10 seconds. Here, when displaying one test problem set on display device 10, PC 30 sequentially displays on display device 10, as illustrated in FIG. 10, fade-in image p8 that provides a fade-in effect, a test image (in this example, second image p2), a correct answer image (in this example, correct answer image p20), and fade-out image p9 that provides a fade-out effect. The display time of fade-in image p8 is, for example, approximately 0.5 seconds, the display time of the test image is, for example, approximately 5 seconds or 7 seconds, the display time of the correct answer image is, for example, approximately 2 seconds, and the display time of fade-out image p9 is, for example, approximately 0.5 seconds. Note that when displaying one test problem set on display device 10, PC 30 need not display fade-in image p8 and fade-out image p9 on display device 10.

Next, PC 30 displays transition image p6 on display device 10 to notify the subject that the task of the Stroop test is changing. Transition image p6 includes a character string (in this example, "The type of task will change") in the center to notify the subject that the task of the Stroop test is changing. Here, when displaying transition image p6, PC 30 sequentially displays on display device 10, as illustrated in FIG. 11, fade-in image p61 that provides a fade-in effect, transition image p6, and fade-out image p62 that provides a fade-out effect. The display time of fade-in image p61 is, for example, approximately 0.5 seconds, the display time of transition image p6 is, for example, approximately 2 seconds, and the display time of fade-out image p62 is, for example, approximately 0.5 seconds. Note that when displaying transition image p6 on display device 10, PC 30 need not display fade-in image p61 and fade-out image p62 on display device 10.

Next, PC 30 displays a test image (third image p3) for the reverse Stroop task on display device 10. Here, PC 30 sequentially displays a plurality of test problem sets (for example, 4 test problem sets) on display device 10, with each test problem set including third image p3 and correct answer image p30. The method of displaying the test problem sets here is the same as the method of displaying the test problem sets in the reverse Stroop control task. PC 30 displays transition image p6 on display device 10. The method of displaying transition image p6 here is the same as the method of displaying transition image p6 described above.

Next, PC 30 displays a test image (second image p2) for the Stroop control task on display device 10. Here, PC 30 sequentially displays a plurality of test problem sets (for example, 4 test problem sets) on display device 10, with each test problem set including second image p2 and correct answer image p20. The method of displaying the test problem sets here is the same as the method of displaying the test problem sets in the reverse Stroop control task. PC 30 displays transition image p6 on display device 10. The method of displaying transition image p6 here is the same as the method of displaying transition image p6 described above.

Next, PC 30 displays a test image (first image p1) for the Stroop task on display device 10. Here, PC 30 sequentially displays a plurality of test problem sets (for example, 4 test problem sets) on display device 10, with each test problem set including first image p1 and correct answer image p10. The method of displaying the test problem sets here is the same as the method of displaying the test problem sets in the reverse Stroop control task. PC 30 displays transition image p6 on display device 10. The method of displaying transition image p6 here is the same as the method of displaying transition image p6 described above.

Finally, PC 30 displays end image p7 on display device 10 to notify the subject of the end of the Stroop test. End image p7 includes a character string (in this example, "The test is now over") in the center to notify the subject of the end of the Stroop test. The display time of end image p7 is, for example, approximately 2 seconds.

Although PC 30 displays the test images on display device 10 in the order of: the test image for the reverse Stroop control task (fourth image p4); the test image for the reverse Stroop task (third image p3); the test image for the Stroop control task (second image p2); and the test image for the Stroop task (first image p1) in the example illustrated in FIG. 9, the present disclosure is not limited to this order. For example, PC 30 may display the test images on display device 10 in the order of: the test image for the Stroop control task (second image p2); the test image for the Stroop task (first image p1); the test image for the reverse Stroop control task (fourth image p4); and the test image for the reverse Stroop task (third image p3).

In the example illustrated in FIG. 9, PC 30 may omit displaying the test image for the Stroop control task (second image p2) and the test image for the reverse Stroop control task (fourth image p4).

In the example illustrated in FIG. 9, when displaying one test problem set on display device 10, PC 30 may omit displaying the correct answer image.

### 6. Verification Data

Hereinafter, verification data for Stroop test system 1 according to the embodiment, i.e., the Stroop test method according to the embodiment, will be explained with reference to FIG. 12 and FIG. 13. FIG. 12 illustrates one example of evaluation information of a subject without impairment in cognitive functions including attention (i.e., a healthy subject). FIG. 13 illustrates one example of evaluation information of a subject with impairment in cognitive functions including attention (i.e., a subject with cognitive impairment).

Both (a) in FIG. 12 and (a) in FIG. 13 indicate that test results (evaluation information) for the Stroop control task are displayed on display 35. More specifically, in (a) in FIG. 12 and (a) in FIG. 13, second image p2 is displayed on display 35. The distribution map of the subject is also superimposed and displayed on second image p2. Note that although not illustrated in (a) in FIG. 12 and (a) in FIG. 13, the fixation rate of the subject for the correct choice is superimposed and displayed on second image p2.

Both (b) in FIG. 12 and (b) in FIG. 13 indicate that test results (evaluation information) for the Stroop task are displayed on display 35. More specifically, in (b) in FIG. 12 and (b) in FIG. 13, first image p1 is displayed on display 35. The distribution map of the subject is also superimposed and displayed on first image p1. Note that although not illustrated in (b) in FIG. 12 and (b) in FIG. 13, the fixation rate of the subject for the correct choice and the fixation rate of the subject for the distractor choice may be superimposed and displayed on first image p1.

In FIG. 12 and FIG. 13, in the distribution map of the subject superimposed and displayed on the test image (here, first image p1 or second image p2), heat map information is represented by the density of dots. Stated differently, the higher the density of dots, the more concentrated the gaze point is indicated to be.

As illustrated in (a) in FIG. 12 and (a) in FIG. 13, both healthy subjects and subjects with cognitive impairment have their gaze points concentrated on the correct choice (here, choice p221), and are selecting the correct answer for the Stroop control task. Both healthy subjects and those with cognitive impairment demonstrate a high fixation rate for the correct choice (91.3% and 90.9%, respectively) in the Stroop control task, reaching the correct answer without hesitation. Here, the subject (here, both healthy subjects and those with cognitive impairment) reaching the correct answer for the task without hesitation being quantitatively expressed as a high fixation rate is important for the evaluation of the subject in the Stroop test. Therefore, referring to the evaluation information illustrated in (a) in FIG. 12 and (a) in FIG. 13, it can be seen that both healthy subjects and those with cognitive impairment are able to correctly recognize the color of the ink.

As illustrated in (b) in FIG. 12, healthy subjects have their gaze points concentrated on the correct choice (here, choice p123), and are selecting the correct answer for the Stroop task. Moreover, healthy subjects are not fixating at all on the distractor choice (here, choice p122). Focusing on the fixation rate of healthy subjects, the fixation rate for the correct choice is 95.2% and 0% for the distractor choice, indicating the healthy subjects reached the correct answer for the Stroop task without hesitation. Therefore, referring to the evaluation information illustrated in (b) in FIG. 12, it can be seen that healthy subjects are hardly affected by the Stroop effect, i.e., interference from the text. Here, the subject (here, healthy individuals) being hardly affected by interference from text being quantitatively expressed as a low fixation rate for the distractor choice is important for the evaluation of the subject in the Stroop test.

However, as illustrated in (b) in FIG. 13, subjects with cognitive impairment are hardly fixating on the correct choice, and are not selecting the correct answer for the Stroop task. Also, the gaze points of subjects with cognitive impairment are concentrated on the distractor choice. Focusing on the fixation rate of subjects with cognitive impairment, the fixation rate for the correct choice is 0.8% and 91.8% for the distractor choice. Therefore, referring to the evaluation information illustrated in (b) in FIG. 13, it can be seen that subjects with cognitive impairment are strongly affected by the Stroop effect, i.e., interference from the text. Here, the subject (here, those with cognitive impairment) being strongly affected by interference from text being quantitatively expressed as a high fixation rate for the distractor choice is important for the evaluation of the subject in the Stroop test.

As illustrated in (b) in FIG. 12 and (b) in FIG. 13, focusing on the test problem area (here, test problem area p11), healthy subjects are fixating on the character string of the question (here, the character string "ink color" that is underlined), whereas subjects with cognitive impairment are fixating on the character string indicating the color name (here, the character string "green" displayed in a relatively large font). This also shows that subjects with cognitive impairment are strongly affected by the Stroop effect, i.e., interference from the text.

As described above, the evaluation information can be used to quantitatively evaluate the attention and judgment of the subject. More specifically, in the evaluation information, the degree of correctness and the degree of influence of the Stroop effect within a single test problem are quantitatively expressed as continuous variables. For example, in conventional Stroop tests, the evaluation obtained from a single test problem is only a discrete binary value of 1 point if correct or 0 points if incorrect. Therefore, in order to evaluate the degree of correctness and the degree of influence of the Stroop effect, it is necessary to present a large number of test problems to the subject. In contrast, in the above-described evaluation information, the degree of correctness and the degree of influence of the Stroop effect can be quantitatively expressed as continuous variables. Therefore, compared to conventional Stroop tests, the number of test problems presented to the subject can be reduced, and the time required for the Stroop test can be shortened. In the above-described evaluation information, within a single test problem, it is possible to objectively and quantitatively evaluate not only the correct answer but also the content and degree of interference (incorrect answer).

Next, a comparison between Stroop test system 1 according to the embodiment, i.e., the Stroop test method according to the embodiment, and a conventional Stroop test will be explained with reference to FIG. 14. In (a) in FIG. 14, the vertical axis indicates subject fixation rate on the correct choice in the Stroop test according to the embodiment, and the horizontal axis represents subject score in a conventional Stroop test. In (b) in FIG. 14, the vertical axis indicates subject fixation rate on the distractor choice in the Stroop test according to the embodiment, and the horizontal axis represents subject score in a conventional Stroop test. In (c) in FIG. 14, the vertical axis indicates subject fixation rate on the test problem area in the Stroop test according to the embodiment, and the horizontal axis represents subject score in a conventional Stroop test. In each of (a) to (c) in FIG. 14, the dashed line represents the regression line, "R" indicates the correlation coefficient, and "P" represents the P-value.

Here, the total number of subjects is 30, consisting of 20 healthy subjects and 10 subjects with cognitive impairment. In the Stroop test according to the embodiment, the display time of the test image is 7 seconds, the display time of the correct answer image is 1 second, and the display time of the transition image is 1 second. In the Stroop test according to the embodiment, the results show the outcomes for a total of 4 Stroop tasks, while the results of the conventional Stroop test indicate the outcomes for a total of 60 Stroop tasks in the new Stroop test I. Subject score in the conventional Stroop test represents the number of correct answers out of the total 60 questions.

As illustrated in (a) in FIG. 14, subject fixation rate on the correct choice in the Stroop test according to the embodiment has a positive correlation with subject score in a conventional Stroop test, increasing as the score in the conventional Stroop test increases. As illustrated in (b) in FIG. 14, subject fixation rate on the distractor choice in the Stroop test according to the embodiment has a negative correlation with subject score in a conventional Stroop test, decreasing as the score in the conventional Stroop test increases. As illustrated in (c) in FIG. 14, subject fixation rate on the test problem area in the Stroop test according to the embodiment has a negative correlation with subject score in a conventional Stroop test, decreasing as the score in the conventional Stroop test increases.

As described above, it can be said that the Stroop test according to the embodiment is similarly effective to the conventional Stroop test, despite presenting fewer test problems to the subject. Moreover, in the Stroop test according to the embodiment, it is possible to evaluate parameters that cannot be evaluated in the conventional Stroop test, such as the fixation rate for the test problem area.

### 7. Advantages

Hereinafter, advantages of Stroop test system 1 according to the embodiment, i.e., advantages of the Stroop test method according to the embodiment, will be described.

First, issues with the conventional Stroop test will be explained again. In the conventional Stroop test, with oral or written responses, it is only possible to record an evaluation of correct or incorrect for each individual task of the subject, in other words, an evaluation expressed as qualitative discrete values of 0 or 1. Stated differently, in the conventional Stroop test, it is not possible to record information on the thought process by which the subject arrived at a correct or incorrect answer, or an evaluation expressed as continuous values.

Here, information on the thought process by which a correct or incorrect answer was reached is essential information reflecting the degree of information interference in the Stroop effect. However, in the conventional Stroop test based on existing interview methods, it is only possible to indirectly estimate such information from the required time or the proportion of correct or incorrect answers. Therefore, in the conventional Stroop test, in order to obtain an accurate evaluation, it becomes necessary to present many test problems to the subject, resulting in a significant burden on both the subject and the examiner.

In contrast, in Stroop test system 1 according to the embodiment, i.e., the Stroop test method according to the embodiment, by using eye-tracking technology, gaze position information indicating the position of the subject's gaze on the test image for the Stroop test is obtained, and evaluation information indicating an evaluation of the subject is generated based on the obtained gaze position information. Therefore, in the Stroop test according to the embodiment, it becomes possible to reflect the subject's thought process (how much they hesitated between the correct choice and incorrect choice) during the display of the test image as the degree of fixation on each choice, making it possible for the first time to express the Stroop effect as an objective continuous value. In the Stroop test according to the embodiment, since information such as the fixation rate for the correct choice or the fixation rate for the distractor choice can be simultaneously obtained from a single task, it is possible to quantitatively evaluate the influence of the Stroop effect with relatively few test problems. Therefore, in the Stroop test according to the embodiment, compared to conventional Stroop tests, it is possible to reduce the burden on both the subject and the examiner.

In conventional Stroop tests that involve written responses, how to allocate the response time per question was left to the subject, but in the Stroop test according to the embodiment, the test image forcibly transitions to the next test image at predetermined time intervals. Therefore, in the Stroop test according to the embodiment, there is an advantage that it is possible to quantitatively evaluate whether the subject was able to select the correct choice within the predetermined time, i.e., within the response time allocated per question.

As described above, Stroop test system 1 according to the embodiment, i.e., the Stroop test method, has the advantage that the test can be performed easily and a quantitative evaluation can be readily obtained. If the Stroop test according to the embodiment enables more convenient and quantitative evaluation of the Stroop effect, it is expected to be widely utilized in clinical and healthcare fields as a convenient examination method for cognitive impairment, including dementia.

In the embodiment, the test image is rectangular in shape, and a plurality of choices in the response area are respectively positioned at a plurality of corners (i.e., the four corners). Therefore, in the embodiment, since the distance from the test problem area to each choice is equal, in other words, the position of each choice with respect to the test problem area is equivalent, there is an advantage that it becomes easier to evaluate the subject more accurately. Here, the distance from the test problem area to the choice affects the time it takes for the subject's gaze to reach the choice from the test problem area, and consequently, affects the fixation rate within the limited time after reaching the choice. For example, the fixation rate of the correct choice indirectly includes the time it takes for the subject's gaze to reach the correct choice, and this time depends on the distance from the test problem area to the correct choice. Moreover, there may be cases where there is a bias in horizontal and vertical eye movements, and such cases are not limited to subjects with cognitive impairments including attention deficits; even healthy individuals may have gaze habits. Therefore, making the distance from the test problem area to each choice equal has the advantage that it becomes easier to evaluate the fixation rate for each choice equally.

By arranging each choice as described above, even in cases where the accuracy of detecting the subject's gaze by eye tracking is relatively low, making the distance between the test problem area and each choice as long as possible has the advantage of making incorrect evaluation of the subject's response based on their gaze less likely.

In the embodiment, the correct choice and the distractor choice are located diagonally opposite each other across the test problem area. Therefore, the embodiment has the advantage that it is possible to reduce the possibility of the subject unconsciously (accidentally) looking at another choice while directing their gaze to either the correct choice or the distractor choice. For example, when the subject directs their gaze to the distractor choice, since they are intentionally directing their gaze believing it to be correct, there is an advantage that by reducing the possibility of unconsciously directing their gaze to the correct choice, it becomes easier to evaluate that the subject is being led to the distractor choice.

In the embodiment, the evaluation information includes information indicating the degree to which the subject fixated on the correct choice (here, the fixation rate for the correct choice). Therefore, the embodiment has the advantage that when repeatedly presenting the same type of test problem for a single task to the subject, it is possible to grasp the improvement in the fixation rate for the correct choice of the subject. In the embodiment, by focusing on the subject's fixation rate for the correct choice in the test problem immediately after the switching which task is presented to the subject, there is also an advantage that it becomes easier to accurately evaluate the influence of the Stroop effect on the subject. This is because the influence of the Stroop effect on the subject, i.e., the interference from text or color, becomes strongest in the test problem immediately after switching the task.

In conventional Stroop tests, practice test problems are presented to the subject each time the task is switched; however, in the Stroop test according to the embodiment, practice test problems are not presented to the subject. Therefore, compared to when practice test problems are presented to the subject, the embodiment also has an advantage that it becomes easier to more accurately evaluate the influence of the Stroop effect on the subject by focusing on the test problem immediately after switching the task. Note that in the Stroop test according to the embodiment, practice test problems may be presented to the subject each time the task is switched, similar to conventional Stroop tests.

In the embodiment, the evaluation information includes information indicating the degree to which the subject fixated on the distractor choice (here, the fixation rate for the distractor choice). Therefore, the embodiment has the advantage that it is possible to quantify the degree to which the subject was led to the distractor choice, in other words, the degree to which they were affected by the Stroop effect. In this way, in the embodiment, since the evaluation information includes the above-described two types of information, there is an advantage that it becomes easier to evaluate the attention for correctly understanding the test problem and the attention for fixating on the correct answer after understanding the test problem.

### 8. Stroop Test Image Generation Method

Next, a Stroop test image generation method according to the embodiment will be explained. The Stroop test image generation method is a method for generating a test image for the Stroop test. The test image generated by the Stroop test image generation method is stored in storage 32 as test image data 300.

FIG. 15 is a flowchart illustrating one example of the Stroop test image generation method according to the embodiment. The Stroop test image generation processes illustrated in FIG. 15 are processes realized by PC 30 executing Stroop test image generation program 312. Here, the Stroop test image generation method generates a plurality of sets of test images for the Stroop task, a plurality of sets of test images for the Stroop control task, a plurality of sets of test images for the reverse Stroop task, and a plurality of sets of test images for the reverse Stroop control task. Each of the plurality of sets includes a plurality of test problems, in other words, a plurality of test images. The Stroop test image generation method illustrated in FIG. 15 illustrates a basic example of generating a set of test images for one of these tasks.

First, PC 30 determines the correct choice for each test problem (S201). Next, PC 30 determines the distractor choice for each test problem (S202). In step S202, PC 30 determines the distractor choice for each test problem such that it differs from the correct choice and the correct choice of the next test problem. Next, PC 30 determines the position of the correct choice in the test image for each test problem (S203). In step S203, for each test problem, PC 30 determines the position of the correct choice to be the vertically opposite position of the correct choice in the previous test problem. Next, PC 30 determines the position of the distractor choice in the test image for each test problem (S204). In step S204, for each test problem, PC 30 determines the position of the distractor choice to be the vertically opposite position of the correct choice. Then, PC 30 determines the positions of the remaining incorrect choices in the test image for each test problem (S205). This generates a test image set for one task.

Hereinafter, specific examples of the Stroop test image generation method according to the embodiment will be given. Here, an example of generating the test image data illustrated in FIG. 16 through FIG. 19 will be given. FIG. 16 illustrates one example of test image data for a Stroop task generated by the Stroop test image generation method according to the embodiment. In FIG. 16, "Ink color (correct answer)" represents the correct choice, and "Text (interference)" represents the distractor choice. FIG. 17 illustrates one example of test image data for a Stroop control task generated by the Stroop test image generation method according to the embodiment. FIG. 18 illustrates one example of test image data for a reverse Stroop task generated by the Stroop test image generation method according to the embodiment. In FIG. 18, "Text (correct answer)" represents the correct choice, and "Ink color (interference)" represents the distractor choice. FIG. 19 illustrates one example of test image data for a reverse Stroop control task generated by the Stroop test image generation method according to the embodiment.

Each unit of test image data includes two series (here, series A and series B). Each series includes four sets. Each set includes four test problems (that is, test images). Therefore, the Stroop test image generation method according to the embodiment generates a total of 32 test images for one task, and generates a total of 128 test images for four tasks. Note that the number of series, the number of sets included in one series, and the number of test problems (that is, test images) included in one set may be set arbitrarily.

Hereinafter, an example of generating a test image set for the Stroop task will be given. First, PC 30 generates a first set of test images for the Stroop task. PC 30 determines the initial parameter, namely the correct choice (in this case, the correct ink color) for the first question. PC 30 determines the initial parameter by, for example, accepting an input of the initial parameter made by an operator via inputter 33. Alternatively, PC 30 may randomly determine the initial parameter by itself. Next, PC 30 determines the correct choice for the second question, the correct choice for the third question, and the correct choice for the fourth question alternately such that the number of characters in the correct choice is either 2 or 3 characters.

Next, PC 30 determines the distractor choice for each test problem. Here, PC 30 determines the ink color of the distractor choice for each test problem to be different from the ink color of the correct choice, and different from the ink color of the correct choice for the next test problem, and essentially to have a number of characters different from the number of characters of the correct choice. Note that when there is no ink color that satisfies the third condition, PC 30 may determine an ink color with the same number of characters as the correct choice as the ink color of the distractor choice.

Next, PC 30 determines the position of each choice in the test image for each test problem. First, PC 30 randomly determines the position of the correct choice for the first question. Next, PC 30 determines the position of the correct choice for the second and subsequent questions to be on either the left or right side vertically opposite to the position of the correct choice in the previous question, and so as not to overlap with the position of the correct choice for other test problems in the same set. Note that PC 30 places an incorrect choice with an ink color having a number of characters different from the number of characters of the correct choice adjacent to the correct choice in the left-right direction, and if possible, so as not to overlap with the incorrect choice in the previous question.

PC 30 determines the position of the distractor choice for each test problem to be on either the left or right side vertically opposite to the position of the correct choice, and so as not to overlap with the position of the distractor choice for other test problems in the same set. In this manner, PC 30 generates a first set of test images for the Stroop task.

Next, PC 30 generates a second set of test images for the Stroop task. First, PC 30 determines the correct choice for each test problem. PC 30 determines the correct choice for the first question so as not to have appeared as the correct choice in at least the previous question and the question before that, and so as not to overlap with the correct choice for the first question in the previous set. Next, PC 30 determines the correct choice for the second question so as not to have appeared as the correct choice in at least the previous question and the question before that, and so as not to overlap with the correct choice for the first question. Next, PC 30 determines the correct choice for the third question and the correct choice for the fourth question so as not to overlap with the order of the correct choice for the third question and the correct choice for the fourth question, respectively, in the previous set.

Next, PC 30 determines the distractor choice for each test problem. Here, PC 30 determines the ink color of the distractor choice for each test problem to be different from the ink color of the correct choice, and different from the ink color of the correct choice for the next test problem, and essentially to have a number of characters different from the number of characters of the correct choice. Note that when there is no ink color that satisfies the third condition, PC 30 may determine an ink color with the same number of characters as the correct choice as the ink color of the distractor choice.

Next, PC 30 determines the position of each choice in the test image for each test problem. Here, PC 30 determines the position of the correct choice for the first question to be adjacent in the left-right direction to that of the second question in the previous set (first set). Next, PC 30 determines the position of the correct choice for the second question to be adjacent in the left-right direction to that of the second question in the previous set (first set). Next, PC 30 determines the position of the correct choice for the third question to be adjacent in the left-right direction to that of the third question in the previous set (first set). Then, PC 30 determines the position of the correct choice for the fourth question to be adjacent in the left-right direction to that of the fourth question in the previous set (first set). Stated differently, for each test problem, PC 30 determines the position of the correct choice to be the vertically opposite position of the correct choice in the previous test problem. As used herein, "previous question" corresponds to the fourth question of the previous set in the case of the question preceding the first question. Note that PC 30 places an incorrect choice with an ink color having a number of characters different from the number of characters of the correct choice adjacent to the correct choice in the left-right direction, and if possible, further so as not to overlap with the incorrect choice in the previous question.

PC 30 determines the position of the distractor choice for each test problem to be on either the left or right side vertically opposite to the position of the correct choice, and so as not to overlap with the position of the distractor choice for the same test problem number in the previous set. For example, PC 30 determines the position of the distractor choice for the first question so as not to overlap with the position of the distractor choice for the first question in the first set. In this manner, PC 30 generates a second set of test images for the Stroop task.

Next, PC 30 generates a third set of test images for the Stroop task. First, PC 30 determines the correct choice for each test problem. PC 30 determines the correct choice for the first question to be the correct choice for the first question in the first set. Next, PC 30 determines the correct choice for the second question so as not to have appeared as the correct choice in at least the previous question and the question before that, and so as not to overlap with the correct choice for the second question in the first set. Next, PC 30 determines the correct choice for the third question to be a color with the same number of characters as the correct choice for the second question. PC 30 determines the ink color of the correct choice for the fourth question to be an ink color that has not yet been used in the same set.

Next, PC 30 determines the distractor choice for each test problem. Here, PC 30 determines the ink color of the distractor choice for each test problem to be different from the ink color of the correct choice, and different from the ink color of the correct choice for the next test problem. PC 30 determines the distractor choice for the first question to be an ink color with the same number of characters as the correct choice. Note that when there is no ink color that satisfies the third condition, PC 30 may determine an ink color with the same number of characters as the correct choice as the ink color of the distractor choice.

Next, PC 30 determines the position of each choice in the test image for each test problem. Here, PC 30 determines the position of the correct choice for the first question to be vertically opposite to the position of the correct choice for the first question in the first set, and so as not to overlap with the position of the fourth question in the previous set (second set). Next, PC 30 determines the position of the correct choice for the second question to be vertically opposite to that of the first question, while maintaining the same horizontal position. Next, PC 30 determines the position of the correct choice for the third question to be adjacent in the left-right direction to that of the first question. Stated differently, this position is the vertically opposite position of the correct choice in the second question. PC 30 determines the position of the correct choice for the fourth question to be a position that has not yet been used in the same set. Note that PC 30 places an incorrect choice with an ink color having a number of characters different from the number of characters of the correct choice adjacent to the correct choice in the left-right direction, and if possible, further so as not to overlap with the incorrect choice in the previous question.

PC 30 determines the position of the distractor choice for the first question to be on either the left or right side vertically opposite to the position of the correct choice, and so as not to overlap with the position of the distractor choice for the fourth question in the second set. Next, PC 30 determines the position of the distractor choice for the second question to be diagonally opposite the correct choice across the test problem area. Next, PC 30 determines the position of the distractor choice for the third question to be adjacent in the left-right direction to that of the first question. PC 30 determines the position of the distractor choice for the fourth question to be a position that has not yet been used in the same set. In this manner, PC 30 generates a third set of test images for the Stroop task.

Next, PC 30 generates a fourth set of test images for the Stroop task. First, PC 30 determines the correct choice for each test problem. PC 30 determines the correct choice for the first question so as not to have appeared as the correct choice in at least the previous question and the question before that, and so as not to overlap with the correct choice for the first question in the previous set. Next, PC 30 determines the correct choice for the second question so as not to have appeared as the correct choice in at least the previous question and the question before that, and so as not to overlap with the correct choice for the first question. Next, PC 30 determines the correct choice for the third question and the correct choice for the fourth question so as not to overlap with the order of the correct choice for the third question and the correct choice for the fourth question, respectively, in the previous set.

Next, PC 30 determines the distractor choice for each test problem. Here, PC 30 determines the ink color of the distractor choice for each test problem to be different from the ink color of the correct choice, and different from the ink color of the correct choice for the next test problem. PC 30 determines the distractor choice for the first question to be an ink color with the same number of characters as the correct choice. Note that when there is no ink color that satisfies the third condition, PC 30 may determine an ink color with the same number of characters as the correct choice as the ink color of the distractor choice.

Next, PC 30 determines the position of each choice in the test image for each test problem. Here, PC 30 determines the position of the correct choice for the first question to be adjacent in the left-right direction to that of the first question in the previous set (third set). Next, PC 30 determines the position of the correct choice for the second question to be adjacent in the left-right direction to that of the second question in the previous set (third set). Next, PC 30 determines the position of the correct choice for the second question to be adjacent in the left-right direction to that of the first question. PC 30 determines the position of the correct choice for the fourth question to be a position that has not yet been used in the same set. Stated differently, for each test problem, PC 30 determines the position of the correct choice to be the vertically opposite position of the correct choice in the previous test problem. Note that PC 30 places an incorrect choice with an ink color having a number of characters different from the number of characters of the correct choice adjacent to the correct choice in the left-right direction, and if possible, further so as not to overlap with the incorrect choice in the previous question.

PC 30 determines the position of the distractor choice for the first question to be on either the left or right side vertically opposite to the position of the correct choice, and so as not to overlap with the position of the distractor choice for the first question in the previous set (third set). Next, PC 30 determines the position of the distractor choice for the second question so as not to overlap with the position of the distractor choice for the second question in the previous set (third set). Next, PC 30 determines the position of the distractor choice for the third question to be on either the left or right side vertically opposite to the position of the correct choice, and so as not to overlap with the position of the distractor choice for the first question. PC 30 determines the position of the distractor choice for the fourth question to be a position that has not yet been used in the same set. In this manner, PC 30 generates a fourth set of test images for the Stroop task.

Next, an example of generating a test image set for the Stroop control task will be given. PC 30 replaces the character string indicating the ink color of each choice with a rectangular region filled with that ink color, based on the generated test image set for the Stroop task. In this manner, PC 30 generates first to fourth sets of test images for the Stroop control task.

Next, an example of generating a test image set for the reverse Stroop task will be given. PC 30, based on the generated test image set for the Stroop task, rewrites the "Ink color (correct answer)" column of the task to "Text (correct answer)" and converts the ink color names to black sentence case words, while also rewriting the "Text (interference)" column to "Ink color (interference)" and converting the ink color names to all capital characters representing each color. PC 30 then assigns a background color to each choice according to the name of the ink color. In this manner, PC 30 generates first to fourth sets of test images for the reverse Stroop task. Note that in the Stroop test, the text in the choices are not displayed on display device 10.

Next, an example of generating a test image set for the reverse Stroop control task will be given. PC 30, based on the generated test image set for the reverse Stroop task, removes the "Ink color (interference)" column from the task. In this manner, PC 30 generates first to fourth sets of test images for the reverse Stroop control task.

Note that when PC 30 uses a test image set for any task in the Stroop test, it ensures that the test problem patterns do not overlap with other tasks by, for example, using a different series from those used in other tasks, using a set different from those used in other tasks, or arranging each test problem in reverse order.

Hereinafter, advantages of the Stroop test image generation method according to the embodiment will be described. In the conventional Stroop test, the test problems presented to the subject are fixed, and when the subject repeatedly takes the Stroop test, there is an issue that the subject may memorize the test problems and answers, potentially causing the Stroop test to lose its effectiveness. In contrast, the Stroop test image generation method according to the embodiment can randomly generate a test image set to be used for the Stroop test while following predetermined rules. Therefore, the Stroop test image generation method according to the embodiment has the advantage that even when the subject repeatedly takes the Stroop test, the subject does not memorize the test problems and answers, so the effectiveness of the Stroop test is less likely to be lost.

### Other Embodiments

While the Stroop test system and Stroop test method, as well as the Stroop test image generation method according to the present invention have been described above based on the embodiment, the present invention is not limited thereto. Various modifications to the present embodiment that may be conceived by those skilled in the art, as well as other embodiments resulting from combinations of some elements of the embodiments, are intended to be included within the scope of the present invention as long as these do not depart from the essence of the present invention.

In the above embodiment, the correct choice and the distractor choice need not be located diagonally opposite each other. For example, when a plurality of choices are located in a cross shape across the test problem area, the correct choice and the distractor choice may be located on any of the straight lines forming the cross.

In the above embodiment, the plurality of choices in the response area need not be respectively positioned at a plurality of corners, and it is sufficient if they are positioned in the area surrounding the test problem area. For example, when the test image is circular in shape, the plurality of choices may be positioned at equal intervals on a circumference surrounding the test problem area.

In the above embodiment, the test problem area need not be positioned at the center of the test image. For example, the test problem area may be positioned in the upper half of the test image, and the response area may be positioned in the lower half.

In the above embodiment, the test image need not be limited to a rectangular shape, and may be, for example, a polygonal shape other than rectangular or a circular shape.

In the above embodiment, the predetermined time, which is the interval at which the test image is displayed on display device 10, is fixed; however, the present disclosure is not limited to this. For example, the predetermined time may be determined based on the difficulty level of the Stroop test or the age of the subject. Stated differently, the predetermined time may be variable depending on the subject. For example, when the subject is a young person, the predetermined time may be set relatively short, and when the subject is an elderly person, the predetermined time may be set relatively long. For example, when the difficulty level of the Stroop test is high, the predetermined time may be set relatively short, and when the difficulty level is low, the predetermined time may be set relatively long. In this way, making the predetermined time variable makes it easier to implement a Stroop test that is suitable for each subject, leading to an advantage that an improvement in the accuracy of the Stroop test can be expected.

Here, which difficulty level of Stroop test the subject takes can be determined based on, for example, the fixation rate for the correct choice and the fixation rate for the test problem area in each test when the subject takes a plurality of Stroop tests having mutually different predetermined times. For example, if the fixation rate for the correct choice and the fixation rate for the test problem area in each test are high, the difficulty level of the next Stroop test may be increased. For example, if the fixation rate for the correct choice and the fixation rate for the test problem area in each test are low, the difficulty level of the next Stroop test may be decreased.

The difficulty level of the Stroop test can be changed without changing the predetermined time. For example, when generating evaluation information in evaluator 39, by shortening the time to be evaluated in the distribution map compared to the predetermined time, it is possible to achieve a state similar to when the predetermined time is shortened pseudo-virtually, i.e., a state where the difficulty level of the Stroop test is increased. In such cases, there is an advantage that even without newly implementing a Stroop test with increased difficulty for the subject by shortening the predetermined time or the like, it is possible to evaluate the subject similarly to when a Stroop test with increased difficulty is implemented.

Furthermore, the approach of changing the predetermined time according to the difficulty level of the test or the age of the subject can be applied not only to the Stroop test but also to other tests using eye tracking. Stated differently, the test method may be a test method of indicating an evaluation of a subject's cognitive function based on gaze position information indicating the position of the subject's gaze on a test image presented to the subject for a predetermined time. The predetermined time may be determined based on the difficulty level of the test or the age of the subject. Note that in the test method, a plurality of test images may be sequentially presented to the subject, or a single test image may be presented to the subject.

The Stroop test according to the above embodiment can also be applied to, for example, the "Position Stroop Test". In such cases, in the test problem area of the test image, the words "top", "middle", and "bottom" may be randomly displayed one word each in the upper, middle, and lower sections, and in the response area, the words representing the correct choice and incorrect choices, i.e., "top", "middle", and "bottom" may be displayed. Evaluator 39 may then evaluate whether or not the subject can fixate on the choice indicating the position of the word without being confused by the meaning of the word.

In the above embodiment, the test image is presented to the subject by being displayed on display device 10; however, the present disclosure is not limited to this. For example, the test image may be presented to the subject on paper. In this case as well, it is possible to obtain the trajectory of the subject's gaze on the test image presented on paper by means of eye tracking.

In the above embodiment, the Stroop test image generation method may be at least the following method. The Stroop test image generation method generates at least a plurality of test images that satisfy a predetermined condition. Each of the plurality of test images includes a test problem area that displays a test problem to be given to the subject and a response area that displays a plurality of choices including a correct choice and one or more incorrect choices for selection by the subject to answer the test problem by gazing. The one or more incorrect choices include a distractor choice capable of influencing the subject due to the Stroop effect. The predetermined condition includes that: (1) the test problem area is positioned at the center of the test image, (2) the response area is positioned in the surrounding area of the test problem area, and (3) the distractor choice in the test image is different from the correct choice in the test image and the correct choice in the test image to be presented next to the subject.

### Additional Comments

As described above, the Stroop test method according to a first aspect indicates an evaluation of a subject based on gaze position information indicating a position of a gaze of the subject on a test image (S102 to S105). The test image includes a test problem area that displays a test problem to be given to the subject and a response area that displays a plurality of choices including a correct choice and one or more incorrect choices for selection by the subject to answer the test problem by gazing. The one or more incorrect choices include a distractor choice capable of influencing the subject due to the Stroop effect.

With this, there is an advantage that the test can be easily performed and a quantitative evaluation can be readily obtained.

In the Stroop test method according to a second aspect, in the first aspect, test problem area p11 displays a character string indicating a color name different from the correct color, and a test problem sentence regarding a color used for the character string. The correct choice (choice p121) is a character string indicating the correct color. The distractor choice (choice p124) is a character string indicating the color name in test problem area p11.

With this, there is an advantage that the Stroop task test can be easily performed and a quantitative evaluation can be readily obtained.

In the Stroop test method according to a third aspect, in the first aspect, test problem area p31 displays a character string indicating a color name represented in a color different from the correct color, and a test problem sentence regarding the meaning of the character string. The correct choice (choice p322) is a region filled with the correct color. The distractor choice (choice p321) is a region filled with the color used for the character string indicating the color name in test problem area p31.

With this, there is an advantage that the reverse Stroop task test can be easily performed and a quantitative evaluation can be readily obtained.

In the Stroop test method according to a fourth aspect, in any one of the first to third aspects, the test problem area is positioned at a center of the test image, and the response area is positioned in a surrounding area of the test problem area.

With this, there is an advantage that when the subject's gaze moves toward each choice after fixating on the test problem area, the trajectory of the subject's gaze is more likely to be dispersed, and the trajectories of the subject's gaze toward each choice are less likely to overlap.

In the Stroop test method according to a fifth aspect, in the fourth aspect, the test image is rectangular in shape. The plurality of choices in the response area are respectively positioned at a plurality of corners of the test image.

With this, there is an advantage that the trajectories of the subject's gaze toward each choice are even less likely to overlap.

In the Stroop test method according to a sixth aspect, in the fourth aspect, the correct choice and the distractor choice are located diagonally opposite each other across the test problem area.

With this, it is possible to clearly distinguish between the correct choice and the distractor choice, and there is an advantage that even if there is a deviation in the subject's gaze, it becomes easier to distinguish and detect whether the subject's gaze is directed toward the correct choice or toward the distractor choice.

In the Stroop test method according to a seventh aspect, in any one of the first to sixth aspects, the test image includes a plurality of test images. The plurality of test images are sequentially displayed at intervals of a predetermined time.

With this, there is an advantage that it is possible to quantitatively evaluate whether the subject was able to select the correct choice within the predetermined time, i.e., within the response time allocated per question.

In the Stroop test method according to an eighth aspect, in the seventh aspect, the predetermined time is determined based on a difficulty level of the Stroop test or the age of the subject.

With this, it becomes easier to implement a Stroop test that is suitable for each subject, leading to an advantage that an improvement in the accuracy of the Stroop test can be expected.

In the Stroop test method according to a ninth aspect, in any one of the first to eighth aspects, the evaluation includes information indicating a degree to which the subject fixated on the correct choice and information indicating a degree to which the subject fixated on the distractor choice.

With this, there is an advantage that since information on the degree to which the subject fixated on the correct choice and the degree to which the subject fixated on the distractor choice can be simultaneously obtained from a single task, it is possible to quantitatively evaluate the influence of the Stroop effect with relatively few test problems.

In the Stroop test method according to a tenth aspect, in the ninth aspect, the evaluation further includes information indicating a degree to which the subject fixated on the test problem area.

With this, there is an advantage that it is possible to evaluate parameters that cannot be evaluated in conventional written or oral Stroop tests.

In the Stroop test method according to an eleventh aspect, in any one of the first to tenth aspects, the test image is presented to the subject by being displayed on display device 10.

With this, there is an advantage that it is easier to present the test image to the subject compared to presenting a printed test image on paper to the subject.

The Stroop test program according to a twelfth aspect is for causing a computer to execute: obtaining gaze position information indicating the position of the subject's gaze on the test image; generating evaluation information indicating an evaluation of the subject based on the obtained gaze position information; and outputting the generated evaluation information. The test image includes a test problem area that displays a test problem to be given to the subject and a response area that displays a plurality of choices including a correct choice and one or more incorrect choices for selection by the subject to answer the test problem by gazing. The one or more incorrect choices include a distractor choice capable of influencing the subject due to the Stroop effect.

With this, there is an advantage that the test can be easily performed and a quantitative evaluation can be readily obtained.

Stroop test system 1 according to a thirteenth aspect includes an obtainer (detector 37 and generator 38, or obtainer 40), evaluator 39, and an outputter (display 35). The obtainer obtains gaze position information indicating a position of a gaze of a subject on a test image. Evaluator 39 generates evaluation information indicating an evaluation of the subject based on the gaze position information obtained by the obtainer. The outputter outputs the evaluation information generated by evaluator 39. The test image includes a test problem area that displays a test problem to be given to the subject and a response area that displays a plurality of choices including a correct choice and one or more incorrect choices for selection by the subject to answer the test problem by gazing. The one or more incorrect choices include a distractor choice capable of influencing the subject due to the Stroop effect.

With this, there is an advantage that the test can be easily performed and a quantitative evaluation can be readily obtained.

The Stroop test image generation method according to a fourteenth aspect generates a plurality of test images that satisfy a predetermined condition (S201 to S205). Each of the plurality of test images includes a test problem area that displays a test problem to be given to the subject and a response area that displays a plurality of choices including a correct choice and one or more incorrect choices for selection by the subject to answer the test problem by gazing. The one or more incorrect choices include a distractor choice capable of influencing the subject due to the Stroop effect. The predetermined condition includes that: (1) the test problem area is positioned at the center of the test image, (2) the response area is positioned in the surrounding area of the test problem area, and (3) the distractor choice in the test image is different from the correct choice in the test image and the correct choice in the test image to be presented next to the subject.

Accordingly, there is an advantage that even when the subject repeatedly takes the Stroop test, the subject does not memorize the test problems and answers, so the effectiveness of the Stroop test is less likely to be lost.

In the Stroop test image generation method according to a fifteenth aspect, in the fourteenth aspect, the predetermined condition further includes that (4) in each of the plurality of test images, the correct choice is positioned on an opposite side of the test problem area relative to a position of the correct choice in a test image immediately previously presented to the subject among the plurality of test images.

Accordingly, there is an advantage that even when the subject repeatedly takes the Stroop test, the subject does not memorize the test problems and answers, so the effectiveness of the Stroop test is even less likely to be lost.

In the Stroop test image generation method according to a sixteenth aspect, in the fourteenth or fifteenth aspect, the predetermined condition further includes that (5) in each of the plurality of test images, the distractor choice is positioned on an opposite side of the test problem area relative to a position of the correct choice.

With this, it is possible to clearly distinguish between the correct choice and the distractor choice, and there is an advantage that even if there is a deviation in the subject's gaze, it becomes easier to distinguish and detect whether the subject's gaze is directed toward the correct choice or toward the distractor choice.

In the Stroop test image generation method according to a seventeenth aspect, in any one of the fourteenth to sixteenth aspects, the predetermined condition further includes that (6) the test image is rectangular in shape, and the plurality of choices in the response area are respectively positioned at a plurality of corners of the test image.

With this, there is an advantage that when the subject's gaze moves toward each choice after fixating on the test problem area, the trajectory of the subject's gaze is more likely to be dispersed, and the trajectories of the subject's gaze toward each choice are less likely to overlap.

In the Stroop test image generation method according to an eighteenth aspect, in the seventeenth aspect, the predetermined condition further includes that (7) the correct choice and the distractor choice are located diagonally opposite each other across the test problem area.

With this, it is possible to even more clearly distinguish between the correct choice and the distractor choice, and there is an advantage that even if there is a deviation in the subject's gaze, it becomes even easier to distinguish and detect whether the subject's gaze is directed toward the correct choice or toward the distractor choice.

The Stroop test image generation program according to a nineteenth aspect is for causing a computer to execute generating a plurality of test images that satisfy a predetermined condition. Each of the plurality of test images includes a test problem area that displays a test problem to be given to the subject and a response area that displays a plurality of choices including a correct choice and one or more incorrect choices for selection by the subject to answer the test problem by gazing. The one or more incorrect choices include a distractor choice capable of influencing the subject due to the Stroop effect. The predetermined condition includes that: (1) the test problem area is positioned at the center of the test image, (2) the response area is positioned in the surrounding area of the test problem area, and (3) the distractor choice in the test image is different from the correct choice in the test image and the correct choice in the test image to be presented next to the subject.

Accordingly, there is an advantage that even when the subject repeatedly takes the Stroop test, the subject does not memorize the test problems and answers, so the effectiveness of the Stroop test is less likely to be lost.

The test method according to a twentieth aspect indicates an evaluation of a cognitive function of a subject based on gaze position information indicating a position of the subject's gaze on a test image. The test method includes sequentially presenting the test images to the subject for a predetermined time. The predetermined time is determined based on a test difficulty level or an age of the subject.

With this, it becomes easier to implement a test that is suitable for each subject, leading to an advantage that an improvement in the accuracy of the test can be expected.

### [Industrial Applicability]

The present invention is applicable in systems, methods, and programs for conducting a Stroop test.

### [Reference Signs List]

- 1: Stroop test system
- 10: display device
- 11: display screen
- 20: imaging device
- 21: imager
- 22, 23: camera
- 24: light source unit
- 25, 26: light source
- 30: PC
- 31: processor
- 32: storage
- 33: inputter
- 34: audio outputter
- 35: display
- 36: interface
- 37: detector
- 38: generator
- 39: evaluator
- 40: obtainer
- 300: test image data
- 301: first image data
- 302: second image data
- 303: third image data
- 304: fourth image data
- 310: program
- 311: Stroop test program
- 312: Stroop test image generation program
- 313: gaze point data
- 314: distribution map data
- 315: database

## Claims

1. A Stroop test method of indicating an evaluation of a subject based on gaze position information indicating a position of a gaze of the subject on a test image, wherein
the test image includes:
a test problem area that displays a test problem to be given to the subject; and
a response area that displays a plurality of choices including a correct choice and one or more incorrect choices for selection by the subject to answer the test problem by gazing, and
the one or more incorrect choices include a distractor choice capable of influencing the subject due to a Stroop effect.

2. The Stroop test method according to claim 1, wherein
the test problem area displays a character string indicating a color name different from a correct color, and a test problem sentence regarding a color used for the character string,
the correct choice is a character string indicating the correct color, and
the distractor choice is the character string indicating the color name in the test problem area.

3. The Stroop test method according to claim 1, wherein
the test problem area displays a character string indicating a color name represented in a color different from a correct color, and a test problem sentence regarding a meaning of the character string,
the correct choice is a region filled with the correct color, and
the distractor choice is a region filled with a color used for the character string indicating the color name in the test problem area.

4. The Stroop test method according to any one of claims 1 to 3, wherein
the test problem area is positioned at a center of the test image, and
the response area is positioned in a surrounding area of the test problem area.

5. The Stroop test method according to claim 4, wherein
the test image is rectangular in shape, and
the plurality of choices in the response area are respectively positioned at a plurality of corners of the test image.

6. The Stroop test method according to claim 5, wherein
the correct choice and the distractor choice are located diagonally opposite each other across the test problem area.

7. The Stroop test method according to any one of claims 1 to 3, wherein
the test image comprises a plurality of test images, and
the plurality of test images are sequentially presented to the subject at intervals of a predetermined time.

8. The Stroop test method according to claim 7, wherein
the predetermined time is determined based on a Stroop test difficulty level or an age of the subject.

9. The Stroop test method according to any one of claims 1 to 3, wherein
the evaluation includes information indicating a degree to which the subject fixated on the correct choice and information indicating a degree to which the subject fixated on the distractor choice.

10. The Stroop test method according to claim 9, wherein
the evaluation further includes information indicating a degree to which the subject fixated on the test problem area.

11. The Stroop test method according to any one of claims 1 to 3, wherein
the test image is presented to the subject by being displayed on a display device.

12. A Stroop test program for causing a computer to execute:
obtaining gaze position information indicating a position of a gaze of a subject on a test image;
generating evaluation information indicating an evaluation of the subject based on the gaze position information obtained; and
outputting the evaluation information generated, wherein
the test image includes:
a test problem area that displays a test problem to be given to the subject; and
a response area that displays a plurality of choices including a correct choice and one or more incorrect choices for selection by the subject to answer the test problem by gazing, and
the one or more incorrect choices include a distractor choice capable of influencing the subject due to a Stroop effect.

13. A Stroop test system comprising:
an obtainer that obtains gaze position information indicating a position of a gaze of a subject on a test image;
an evaluator that generates evaluation information indicating an evaluation of the subject based on the gaze position information obtained by the obtainer; and
an outputter that outputs the evaluation information generated by the evaluator, wherein
the test image includes:
a test problem area that displays a test problem to be given to the subject; and
a response area that displays a plurality of choices including a correct choice and one or more incorrect choices for selection by the subject to answer the test problem by gazing, and
the one or more incorrect choices include a distractor choice capable of influencing the subject due to a Stroop effect.

14. A Stroop test image generation method comprising:
generating a plurality of test images that satisfy a predetermined condition, wherein
each of the plurality of test images includes:
a test problem area that displays a test problem to be given to a subject; and
a response area that displays a plurality of choices including a correct choice and one or more incorrect choices for selection by the subject to answer the test problem by gazing,
the one or more incorrect choices include a distractor choice capable of influencing the subject due to a Stroop effect, and
the predetermined condition includes that, for a test image among the plurality of test images:
(1) the test problem area is positioned at a center of the test image;
(2) the response area is positioned in a surrounding area of the test problem area; and
(3) the distractor choice in the test image is different from the correct choice in the test image and the correct choice in a test image to be presented next to the subject among the plurality of test images.

15. The Stroop test image generation method according to claim 14, wherein
the predetermined condition further includes that:
(4) in each of the plurality of test images, the correct choice is positioned on an opposite side of the test problem area relative to a position of the correct choice in a test image immediately previously presented to the subject among the plurality of test images.

16. The Stroop test image generation method according to claim 14 or 15, wherein
the predetermined condition further includes that:
(5) in each of the plurality of test images, the distractor choice is positioned on an opposite side of the test problem area relative to a position of the correct choice.

17. The Stroop test image generation method according to claim 14 or 15, wherein
the predetermined condition further includes that:
(6) the test image is rectangular in shape, and the plurality of choices in the response area are respectively positioned at a plurality of corners of the test image.

18. The Stroop test image generation method according to claim 17, wherein
the predetermined condition further includes that:
(7) the correct choice and the distractor choice are located diagonally opposite each other across the test problem area.

19. A Stroop test image generation program for causing a computer to execute:
generating a plurality of test images that satisfy a predetermined condition, wherein
each of the plurality of test images includes:
a test problem area that displays a test problem to be given to a subject; and
a response area that displays a plurality of choices including a correct choice and one or more incorrect choices for selection by the subject to answer the test problem by gazing,
the one or more incorrect choices include a distractor choice capable of influencing the subject due to a Stroop effect, and
the predetermined condition includes that, for a test image among the plurality of test images:
(1) the test problem area is positioned at a center of the test image;
(2) the response area is positioned in a surrounding area of the test problem area; and
(3) the distractor choice in the test image is different from the correct choice in the test image and the correct choice in a test image to be presented next to the subject among the plurality of test images.

20. A test method of indicating an evaluation of a cognitive function of a subject based on gaze position information indicating a position of a gaze of the subject on a test image, the test method comprising:
presenting the test image to the subject for a predetermined time, wherein
the predetermined time is determined based on a test difficulty level or an age of the subject.
